# EUROPEAN PATENT SPECIFICATION

(11) **EP 0 408 764 B1**
(45) Date of publication and mention of the grant of the patent: **03.05.1995**
(21) Application number: 90901896.2
(22) Date of filing: 17.01.1990
(51) Int. Cl.: C12P 21/02, C07K 14/585, C12N 9/80, C12N 15/53

(54) **PROCESS FOR PRODUCTION OF C-TERMINAL ALPHA-AMIDATED PEPTIDES**
VERFAHREN ZUR HERSTELLUNG VON C-TERMINALEN ALPHA-AMIDIERTEN PEPTIDEN
PROCEDE DE PRODUCTION D'UN PEPTIDE ALPHA-AMIDE A SON EXTREMITE C-TERMINALE

(30) Priority: 17.01.1989 JP 5879/89
(43) Date of publication of application: 23.01.1991
(73) Proprietor: SUNTORY LIMITED, Kita-ku, Osaka-shi, Osaka 530 (JP)
(72) Inventor: TANAKA, Shoji, Hyogo 659 (JP); OHSUYE, Kazuhiro, Osaka 567 (JP); MAGOTA, Koji, Osaka 563 (JP)
(74) Representative: Stoner, Gerard Patrick
(86) International application number: JP9000041
(87) International publication number: WO9008194

(56) References cited:
- EP-A- 0 095 351
- EP-A- 0 197 794
- WO-A-85/00043
- WO-A-87/01729
- Chemical Abstracts, volume 83, no. 13, 29 September 1975, (Columbus, 0hio, US), Moore, Graham J et al, see page 235
- Chemical Abstracts, volume 107, no. 21, 23 November 1987, (Columbus, 0hio, US), Tamaoki, Hidetsune et al, see page 358
- Chemical Abstracts, volume 108, no. 13, 28 March 1988 (Columbus, 0hio, US), see page 492
- Chemical Abstracts, volume 105, no. 21, 24 November 1986 (Columbus, 0hio, US), Mizuno, Kensaku et al, see page 303
- Chemical Abstracts, volume 108, no. 15, 11 April 1988, (Columbus, 0hio, US), Mizuno, Kensaku et al, see page 165
- Chemical Abstracts, volume 109, no. 9, 29 August 1988, (Columbus, 0hio, US), 0hsuye, Kazuhiro et al, see page 175
- Chemical Abstracts, volume 109, no. 15, 10 October 1988, (Columbus, 0hio, US), Mizuno, Kensaku et al, see page 289
- Chemical Abstracts, volume 104, no. 11, 17 March 1986, (Columbus, 0hio, US), Hilsted, Linda et al, see page 77

## Description

The present invention relates to processes for the production of a precursor of a C-terminal α-amidated peptide, to processes for the production of a C-terminal α-amidated peptide, and processes for the production of a C-terminal α-amidating enzyme used for the production of a C-terminal α-amidated peptide.

It is generally known that, after translation from a messenger RNA (mRNA), some kinds of peptides or proteins are modified in eukaryotic cells by an intracellular enzyme to mature to a natural-type peptide or protein (post-translational modification), but since prokaryotic hosts such as E. coli, which are widely used to produce peptides or proteins of eukaryote origin, cannot carry out a post-translational modification of an expressed peptide or protein, it is sometimes difficult to directly produce a eukaryotic peptide or protein by a recombinant DNA technique using prokaryotic host cells.

One post-translational modification characteristic of eukaryotic cells of peptides or proteins is a modification reaction wherein an α-position of a carboxy terminal (C-terminal) of a peptide or protein is amidated, i.e., -COOH is converted to -CONH₂ , and it is known that many physiologically active peptides or proteins have been subjected to such modification, and that in some cases the above-mentioned modification of the peptide or protein is essential to the physiological activity thereof. For example, a conversion of the proline amide residue at the C-terminal of natural-type human calcitonin to a proline residue lowers the physiological activity thereof to 1/1,600 of the original activity.

Because of the importance of clarifying the mechanism of an α-amide formation in tissues, and the promising usefulness of the enzyme for the production of C-terminal α-amidated peptides using, for example, recombinant DNA techniques, many attempts have been made to clarify a detailed mechanism of the biosynthesis of C-terminal α-amidated peptides characteristic to eukaryotic cells. In this clarification, first a structure of a precursor of an amidated peptide is clarified from a cDNA analysis of the amidated peptide, and as a result, a general biosynthesis mechanism of such amidated peptides is understood to be that in which RNA is translated to a precursor of an amidated peptide, which is then amidated at the α-position of the C-terminal thereof by a C-terminal α-amidating enzyme. Note, in the above-mentioned reaction, the precursor of the C-terminal α-amidated peptide as a substrate for a C-terminal α-amidating enzyme is a peptide or protein represented by a general formula R-X-Gly, wherein R represents an amino acid sequence of the N-terminal side of the peptide or protein, X represents an amino acid residue which is to be α-amidated at the C-terminal thereof, and Gly represents a glycine residue.

On the other hand, in porcine pituitary, Bradburg, A.F. et al., Nature 298, 686-688, 1982, first characterized the α-amidating activity of converting a synthetic substrate D-Tyr-Val-Gly to D-Tyr-Val-NH₂ , and demonstrated that the C-terminal glycine in the substrate serves as a nitrogen-donor for α-amidation. Also, Eipper et al., Proc. Natl. Acad. Sci. US, 80, 5144-5148, 1983, reported that the activity of the α-amidating enzyme derived from the pituitary gland of rat requires copper cation, ascorbic acid, and molecular oxygen. Husain, I. et al., FEBS Lett., 152 227-281, 1983; and Kizer, J. S. et al., Proc. Natl. Acad. Sci. US, 81, 3228-3232, 1984, also reported a C-terminal α-amidating enzyme, but did not report a purified enzyme. Recently, Murthy A.S.N. et al., J. Biol. Chem., 261, 1815-1822, 1986, partially purified a C-terminal α-amidating enzyme from the pituitary gland of cattle, and showed that several types of enzymes having different molecular weights and electric charges are present.

Recently, Mizuno et al. succeeded in isolating a C-terminal α-amidating enzyme in a homogeneous and pure form from a skin of Xenopus laevis (see Mizuno, K. et al., Biochem. Biophys. Res Commun. 137, 984-991, 1988, and Japanese Patent Application No. 61-131089) and further succeeded in determining an entire primary amino acid sequence of the C-terminal α-amidating enzyme of a skin of Xenopus laevis origin by obtaining and sequencing cDNA; see Mizuno, K. et al., Biochem. Biophys. Res. Commun. 148, 546-552, 1987.

Our own EP-A-299790 (which is in Article 54(3) prior art relation with the present disclosure, but does not designate Denmark) also describes obtaining Xenopus laevis C-terminal α-amidating enzymes, and furthermore discloses use thereof in converting R-Gly to R-NH₂.

Peptide Chemistry 1987 pp 427-430 (reported in Chemical Abstracts 109; 124771r) describes the cloning and expression cDNA encoding C-terminal α-amidating enzyme AE-I, obtained initially from the skin of Xenopus laevis, and effectiveness of the enzyme to convert Ac-Tyr-Phe-Gly to Ac-Tyr-Phe-NH₂.

On the other hand, Eipper, B. et al., Mol. Endo. 1, 777-790, 1987, cloned cDNA of a C-terminal α-amidating enzyme of the bovine pituitary gland, and demonstrated that the enzyme of the bovine pituitary gland is clearly different from the above-mentioned enzyme of Xenopus laevis origin.

The above-mentioned reports demonstrate that there are many C-terminal α-amidating enzymes of eukaryote origin, and this suggests that each enzyme has a particular substrate specificity different from that of the others; namely, a particular C-terminal α-amidating enzyme is present for a particular α-amidated peptide, although this has not been fully demonstrated as yet.

Considering the biosynthesis mechanism of amidated peptides in eukaryotic cells so far clarified as described above, it is expected that a large amount of an amidated peptide can be produced by a procedure as described below. Namely, first a large amount of a precursor of amidated peptide, represented by the formula R-X-Gly, is economically produced by genetic engineering in a prokaryotic host such as E. coli; next a large amount of C-terminal α-amidating enzyme of eukaryotic cell origin is prepared; and finally, the precursor is converted to a desired amidated peptide using the C-terminal α-amidating enzyme under an optimum condition.

There have been many attempts, based on this concept, to produce amidated peptides. Namely, many reports relating to production of precursors of amidated peptides by genetic engineering have been published. For example, Bennett A.D. et al. (PCT Japanese National Publication No. 60-501391; WO 84/04756) describe a process for the production of human calcitonin precursor (hCT-Gly) from a fused protein comprising an active region of chloramphenicol acetyltransferase (CAT) protein and a human calcitonin precursor expressed in E. coli. According to this procedure, however, 44 mg of the fused protein provides only about 1.1 to 2.0 mg of the human calcitonin precursor (hCT-Gly), and therefore, this procedure does not provide an efficient production of the hCT-Gly.

On the other hand, the present inventors (Japanese Patent Application No. 63-49723; EP 0281418 A2) reported an efficient process for the production of hCT-Gly-Lys-Lys-Arg, which contains a human calcitonin precursor moiety, from a fused protein comprising a protein derived from β-galactosidase and the peptide hCT-Gly-Lys-Lys-Arg expressed in E. coli. Nevertheless, this peptide, per se, is not a substrate for a C-terminal α-amidating enzyme, and therefore must be converted to the precursor hCT-Gly.

Further, regarding the choice and the method to obtain an amount of C-terminal α-amidating enzyme sufficient for industrial use, Eeton M.A.W. et al. (PCT Japanese National Publication No. 63-501541; WO 87/01729) reported the production of human calcitonin using a C-terminal α-amidating enzyme partially purified from the pig pituitary gland. Nevertheless, according to this process, and to other known processes, it is economically difficult to obtain a sufficient amount of the C-terminal α-amidating enzyme for use in the industrial production of amidated peptides. In this connection, the present inventors (Japanese Patent Application No. 62-306867; EP 0299790 A2) developed a process which provides a large amount of C-terminal α-amidating enzyme of Xenopus laevis skin origin and its derivatives by genetic engineering. However, C-terminal α-amidating enzyme of Xenopus laevis origin expressed in E. coli and derivatives thereof exhibit lower activity than that purified from the skin of Xenopus laevis, and therefore C-terminal α-amidating enzyme having a higher activity is desired for the use to produce a large amount of α-amidated peptide.

With regard to the optimum condition for an in-vitro production of an amidated peptide from a derivative thereof by a C-terminal α-amidating enzyme, as described above, this enzyme reaction is dependent upon copper cation (Cu²⁺), molecular oxygen, ascorbic acid, and catalase. However, when human calcitonin is produced from a precursor thereof using a C-terminal α-amidating enzyme partially purified from the pig pituitary gland, under the optimum condition for this enzyme, the solubility of the precursor is not sufficient for efficient enzyme reaction. Therefore, to obtain an efficient in-vitro enzyme reaction, an optimum condition for a desired peptide must be found.

As described above, to economically produce a large amount of an amidated peptide, at least three technical problems must be solved, i.e., (1) an efficient production of a precursor peptide for a desired amidated peptide, (2) production of a sufficient amount of a C-terminal α-amidating enzyme having a high potency, and (3) determination of an optimum condition for an in-vitro conversion of a particular precursor to a desired peptide using the C-terminal α-amidating enzyme. Accordingly, at present, intense research into a resolution of these problems is underway.

Accordingly, the aim of the invention is an economical process for the production of a large amount of amidated peptide precursors represented by the formula R-X-Gly, a process for the production of a large amount of a C-terminal α-amidating enzyme having a high potency, and a process for the production of an amidated peptide under an optimum condition.

More specifically, the present invention provides a process for the production of a peptide or protein represented by the formula (II):

R-X-Gly (II)

wherein X represents any amino acid residue, Gly represents a C-terminal glycine residue, and R represents a remaining portion of the peptide or protein, comprising the step of:
hydrolyzing a peptide or protein represented by the formula (I):

R-X-Gly-B

wherein X represents any amino acid residue, Gly represents a glycine residue, B represents a lysine or arginine residue, an oligopeptide consisting essentially of lysine or arginine residues or a combination of lysine and arginine residues, with carboxypeptidase B.

The present invention also provides a process for the production of human calcitonin, represented by the formula

R-NH₂

in which the -NH₂ group is of the α-amidated C-terminal amino acid of the calcitonin molecule, comprising treating a peptide or protein of the formula

R-Gly

in which Gly represents a C-terminal glycine residue with a C-terminal α-amidating enzyme of human thyroid gland origin or a derivative thereof.

The present invention further provides a process for the production of a peptide or protein represented by the formula (III):

R-X-NH₂ (III

)

wherein X-NH₂ represents a C-terminal α-amidated amino acid and R represents a remaining portion of the peptide or protein, comprising the steps of:
(1) hydrolyzing a peptide or protein represented by the formula (I):

   R-X-Gly-B (I)

   wherein X represents any amino acid residue, Gly represents a glycine residue, B represents a lysine or arginine residue, an oligopeptide consisting essentially of lysine or arginine residues or a combination of lysine and arginine residues, and R represents a remaining portion of the peptide or protein, with carboxypeptidase B to produce an intermediate peptide or protein represented by the formula (II):

   R-X-Gly (II)

   wherein X represents any amino acid residue to be α-amidated, Gly represents a C-terminal glycine residue, and R represents a remaining portion of the peptide and protein; and
(2) treating the intermediate peptide or protein represented by the formula (II) with a C-terminal α-amidating enzyme or a derivative thereof.

The present invention still further provides a process for the production of a C-terminal α-amidating enzyme or a derivative thereof, comprising the steps of
culturing animal cells transfected with a plasmid containing a DNA coding for said enzyme or a derivative thereof, and capable of expressing same; and
recovering the enzyme or a derivative thereof.

The present invention also provides a process for the production of a peptide or protein represented by the formula (II):

R-X-Gly (II)

wherein X represents any amino acid residue, Gly represents a C-terminal glycine residue, and R represents a remaining portion of the peptide or protein, comprising the steps of:
(1) obtaining a fused protein represented by the formula (0):

   P-R-X-Gly-B (0)

   wherein P represent a partner protein of the fused protein, X represents any amino acid residue; Gly represents a glycine residue, B represents a C-terminal lysine or arginine residue, or an oligopeptide consisting essentially of lysine or arginine residues or a combination of lysine and arginine residues, and R represents a remaining portion of the protein, by culturing a host transformed with an expression vector containing a DNA coding for the fused protein;
(2) recovering the fused protein;
(3) cleaving the fused protein by a conventional method to form a partner protein (P) and a peptide or protein represented by the formula (I):

   R-X-Gly-B (I)

   wherein R, X, Gly and B have the same meanings as defined under the formula (0);
(4) separating the peptide or protein (I) from the partner protein (P) on the basis of the difference of the isoelectric points thereof; and
(5) hydrolyzing the peptide or protein (I) with carboxypeptidase B to form the desired peptide or protein (II).

The present invention further provides a process for the production of a peptide or protein represented by the formula (III):

R-X-NH₂ (III)

wherein X-NH₂ represents a C-terminal α-amidated amino acid, and R represents a remaining portion of the peptide or protein comprising the steps of:
(1) obtaining a fused protein represented by the formula (0):

   P-R-X-Gly-B (0)

   wherein P represent a partner protein of the fused protein, X represents any amino acid residue, Gly represents a glycine residue, B represents a C-terminal lysine or arginine residue, or an oligopeptide consisting essentially of lysine or arginine residues or a combination of lysine and arginine residues, and R represents a remaining portion of the protein, by culturing a host transformed with an expression vector containing a DNA coding for the fused protein;
(2) recovering the fused protein;
(3) cleaving the fused protein by a conventional method to form a partner protein (P) and a peptide or protein represented by the formula (I):

   R-X-Gly-B (I)

   wherein R, X, Gly and B have the same meanings as defined under the formula (0);
(4) separating the peptide or protein (I) from the partner protein (P) on the basis of the difference of the isoelectric points thereof; and
(5) hydrolyzing the peptide or protein (I) with carboxypeptidase B to form an intermediate peptide or protein represented by the formula (II):

   R-X-Gly (II)

   wherein X represents any amino acid residue, Gly represents a C-terminal glycine residue, and R represents a remaining portion of the peptide or protein; and
(6) treating the peptide or protein (II) with a C-terminal α-amidating enzyme or a derivative thereof.

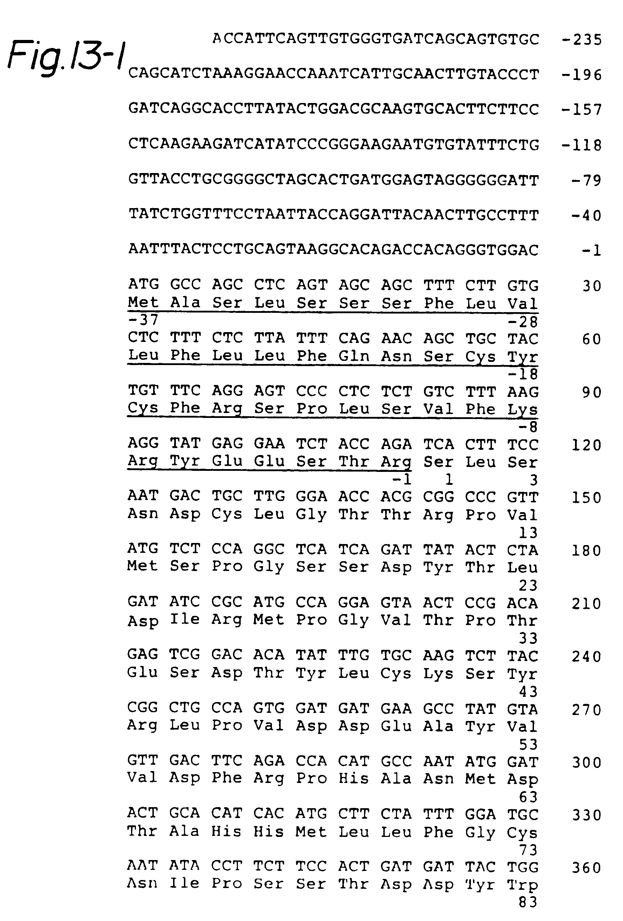

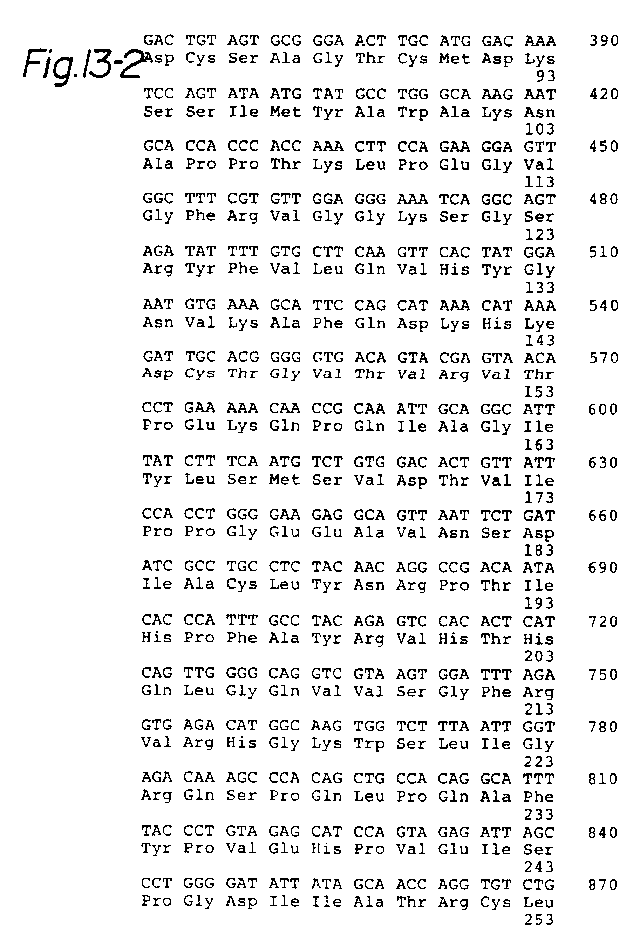

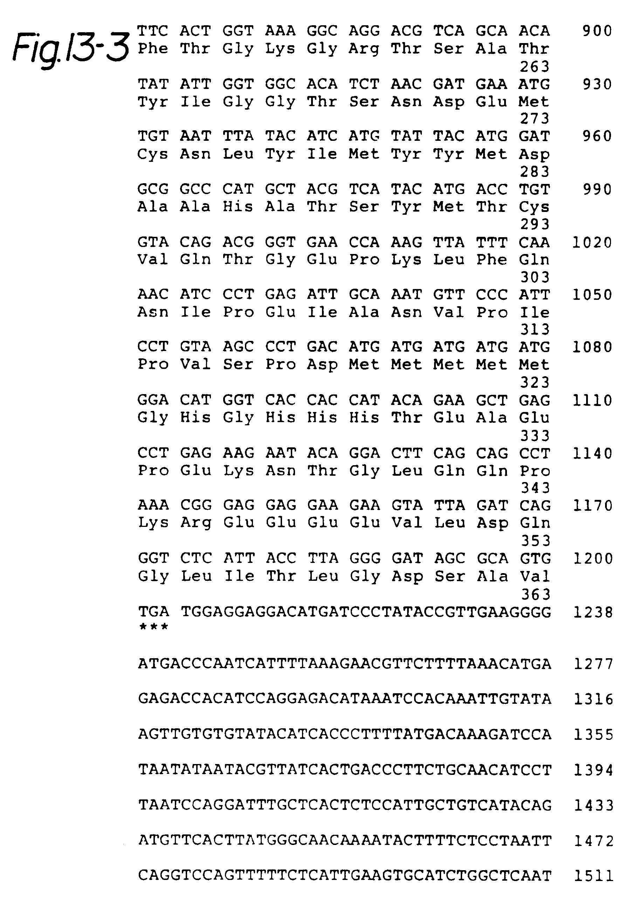

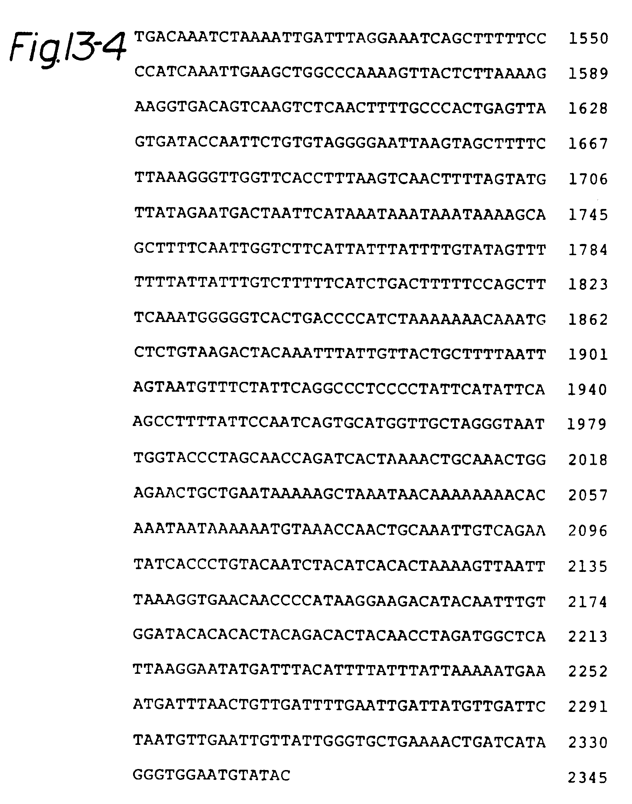

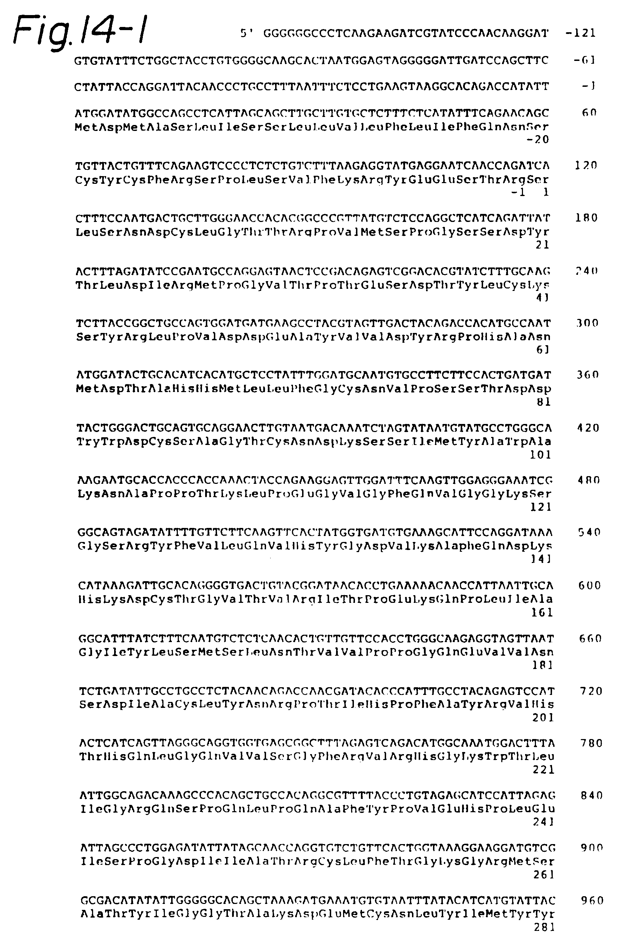

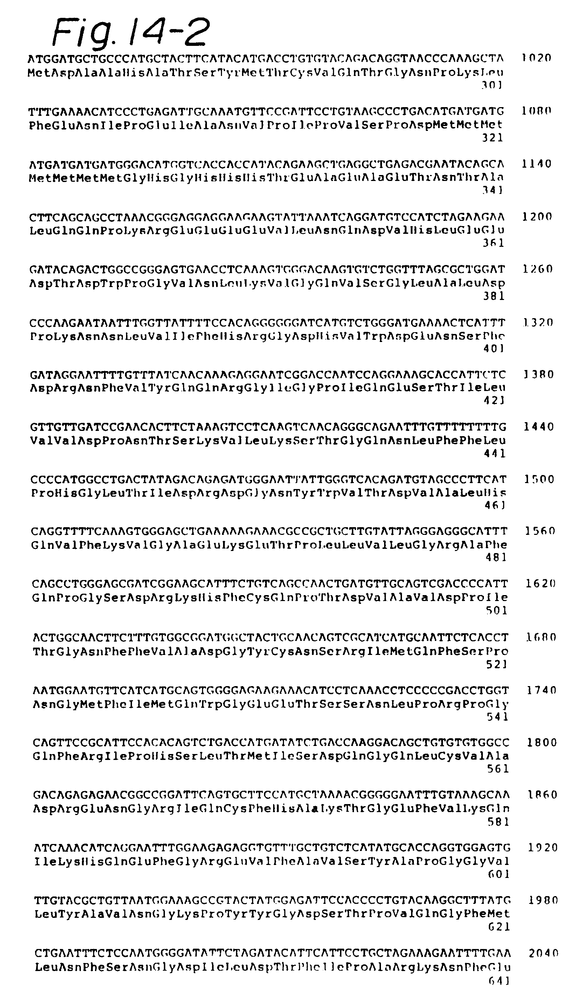

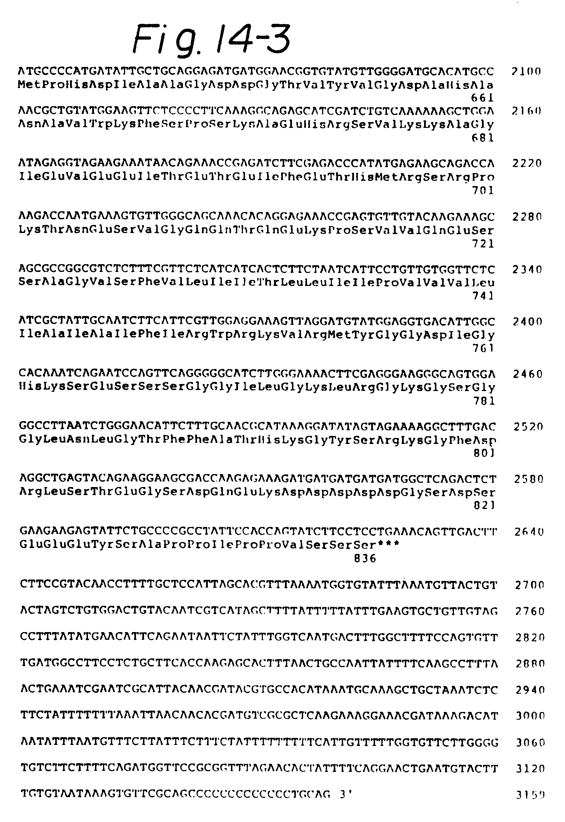

The present invention still further provides a C-terminal α-amidating enzyme having an amino acid sequence starting with the -37th amino acid and terminating at the 363th amino acid shown in Figs. 13-1 to 13-3; and a C-terminal α-amidating enzyme derivative having an amino acid sequence starting with the -39th amino acid and terminating at the 692th amino acid shown in Figs. 14-1 to 14-3.

### BRIEF DESCRIPTION OF THE DRAWING

Figure 1 represents an elution profile of hCH-Gly formed by treating hCT-Gly-Lys-Lys-Arg with carboxypeptidase B(CpB);
Fig. 2 represents a structure of a plasmid pKDPXA457 or pKDPXA799 BglII expressing a C-terminal α-amidating enzyme XA457 or XA799 BglII in animal cells;
Fig. 3 represents purification of the XA799 BglII by ammonium sulfate precipitation;
Fig. 4 is a graph showing an effect of ascorbic acid on an in-vitro amidation of hCT-Gly by the XA799 BglII;
Fig. 5 is a graph showing an effect of a catalase concentration on an in-vitro amidation of hCT-Gly by the XA799 BglII;
Fig. 6 is a graph showing an effect of a copper cation (Cu²⁺) on an in-vitro amidation of hCT-Gly by the XA799 BglII;
Fig. 7 is a graph showing an effect of buffer solutions and the pH thereof on an in-vitro amidation of hCT-Gly by the XA799 BglII;
Fig. 8 is a graph showing an effect of a buffer concentration on an in-vitro amidation of hCT-Gly by the XA799 BglII;
Fig. 9 is a graph showing an effect of a substrate (hCT-Gly) concentration on an in-vitro amidation of hCT-Gly by the XA799 BglII;
Fig. 10 represents elution charts from C18HPLC for samples obtained at 0, 30, and 120 minutes of an in-vitro reaction for a conversion of hCT-Gly to hCT by the XA799 BglII;
Fig. 11 represents a process for the construction of a plasmid having a gene coding for one of three chimeric proteins having different amino acid sequences of basic amino acids;
Fig. 12 represents a result of SDS-polyacrylamide gel electrophoresis showing the productivity of four chimeric proteins having different C-terminal amino acid sequences in E. coli.
Figs. 13-1 to 13-4 represent a nucleotide sequence of a cDNA present in a plasmid pXA457, and an amino acid sequence encoded by the cDNA; and
Figs. 14-1 to 14-3 represent a nucleotide sequence of a cDNA present in a plasmid pXA799, and an amino acid sequence encoded by the cDNA.

### DESCRIPTION OF THE PREFERRED EMBODIMENTS

### (1) Precursor for amidation and process for production thereof

To efficiently prepare a precursor for amidation represented by the formula R-X-Gly, wherein X represents any amino acid residue to be amidated, Gly represents a C-terminal glycine residue, and R represents a remaining portion of the peptide or protein, according to the present invention, a peptide or protein represented by the formula (I):

R-X-Gly-B (I)

wherein X represents any amino acid to be finally amidated, Gly represents a glycine residue, B represents a C-terminal arginine or lysine residue, or an oligopeptide consisting essentially of arginine or lysine residues or a combination of arginine and lysine residues, and R represents a remaining portion of the peptide or protein, is treated by a carboxypeptidase B (CpB).
A precursor of human calcitonin is described as a particular example.

The present inventors reported a process for an efficient production of a large amount of a peptide hCT-Gly-Lys-Lys-Arg containing a human calcitonin precursor moiety (Japanese Patent Application No. 63-49723; EP 0281418 A2), but, this peptide is not a substrate for a C-terminal α-amidating enzyme. Therefore, to obtain a human calcitonin precursor (hCT-Gly), the C-terminal peptide Lys-Ly-Arg must be eliminated from the peptide hCT-Gly-Lys-Lys-Arg. The present inventors found that the human calcitonin precursor hCT-Gly can be extremely efficiently prepared from the peptide hCT-Gly-Lys-Lys-Arg by treating the peptide with carboxypeptidase B (CpB).

Note, this process can be generally applied to the conversion of a peptide, represented by the formula (I), to a precursor for amidation represented by the formula (II), and the peptide (I) can be prepared by chemical synthesis or genetic engineering according to a conventional procedure.

The above-described process for the production of a precursor for amidation is superior to the conventional process wherein the precursor R-X-Gly is produced directly as a fused protein using an E. coli host.

For example, the human calcitonin precursor hCT-Gly is a peptide consisting of 33 amino acid residues including an aspartic acid residue and a lysine residue, and therefore, exhibits an isoelectric point (pI) at an approximately neutral point. Accordingly, it is difficult to separate the precursor from a partner protein after cleavage of the fused protein by a single ion-exchange column chromatography, because in most cases, the hCT-Gly has a neutral isoelectric point. Conversely, since the precursor hCT-Gly-Lys-Lys-Arg has a basic pI, it can be easily separated from a partner protein which forms a fused protein by a simple ion exchange chromatography. Moreover, the precursor hCT-Gly, which has been formed by cleavage of the peptide hCT-Gly-Lys-Lys-Arg with CpB, can be easily separated from possible by-products hCT-Gly-Lys and hCT-Gly-Lys-Lys, as well as the starting material hCT-Gly-Lys-Lys-Arg, by a simple ion exchange chromatography, because the precursor hCT-Gly has a neutral pI but the by-products and the starting material have a basic pI.

As a second advantage of the present process wherein the precursor hCT-Gly is prepared from the peptide hCT-Gly-Lys-Lys-Arg, it was surprisingly found that a fused protein comprising a part of β-galactosidase and hCT-Gly-Lys-Lys-Arg is produced in a remarkably high yield, in comparison with a fused protein comprising a part of β-galactosidase and hCT-Gly, in an E. coli host. Moreover, it was surprisingly found that a fused protein comprising hCT-Gly-Arg, hCT-Gly-Arg-Arg or the like can be produced in a high yield comparable to the fusion protein comprising the hCT-Gly-Lys-Lys-Arg. Accordingly, the present process is remarkably advantageous over a conventional process wherein the precursor hCT-Gly is expressed as a fused protein in which the hCT-Gly is linked to a partner protein.

Note that, as is clear from the above, the B in the formula R-X-Gly-B may be any basic amino acid such as arginine or lysine, or any oligopeptide consisting essentially of two to about ten basic amino acid residues, such as arginine residues or lysine residues, or any combination of arginine and lysine residues.

### (2) Process for production of C-terminal α-amidating enzyme

The present inventors have succeeded in expressing a C-terminal α-amidating enzyme of the Xenopus laevis skin origin, and derivatives thereof, in an E. coli host in a large amount (Japanese Patent Application No. 62-306867; EP 0299790 A2). It was found however, that in this process the C-terminal α-amidating enzyme and derivatives thereof are denatured in E. coli cells and therefore, do not exhibit a C-terminal α-amidating activity. Accordingly, an inactive form of the thus-prepared enzyme should be renatured. This problem was partially solved by a renaturation of the inactive form by treatment with a denaturating agent such as urea or guanidine hydrochloride, followed by renaturation, but, this process provided a renatured enzyme exhibiting an activity lower than that of a native enzyme extracted from the Xenopus laevis skin. The present inventors consider that the reason for the above-mentioned lower activity of the enzyme produced in E. coli is as follows: since the C-terminal α-amidating enzyme of the Xenopus laevis skin origin, and derivatives thereof, has many cysteine residues (for example, a native enzyme of the Xenopus laevis skin origin has 10 cysteine residues), where these proteins are expressed in E. coli cells they cannot form correct disulfide linkages which are the same as those in the native enzyme. Accordingly, the present inventors attempted to enhance the enzyme activity by treating the enzyme expressed in E. coli with a reducing agent such as dithiothreitol or 2-mercaptoethanol, in combination with the above-mentioned denaturating agent, and then oxidizing the reduced protein, but this method failed to enhance the enzyme activity.

To eliminate the above-mentioned difficulty, the present invention provides a process for the production of a C-terminal α-amidating enzyme, or derivatives thereof, wherein the enzyme, or derivative thereof, is expressed in eukaryotic cells such as animal cells.

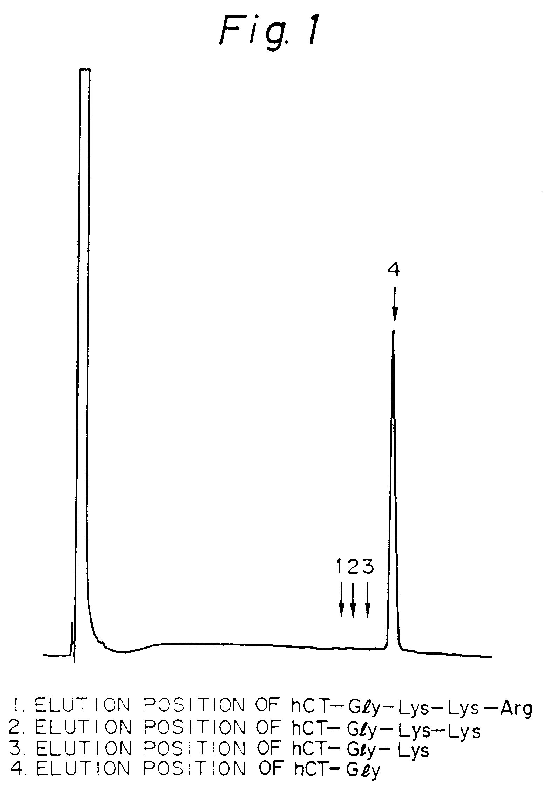

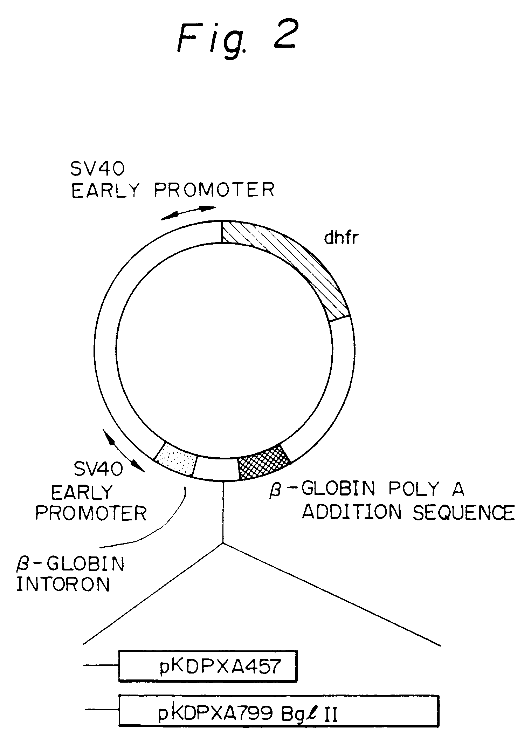

DNAs coding for two types of C-terminal α-amidating enzymes (XA457 and XA799) of the Xenopus laevis origin, have been cloned in plasmids pXA457 and pXA799, respectively (Japanese Patent Application No. 62-306867; EP 0299790 A2). The nucleotide sequence of the cDNA coding for the enzyme XA457, and corresponding amino acid sequence, are described in Figs. 13-1 to 13-4 (Fig. 1-1 to 1-3 of the above patent application), and the nucleotide sequence of the cDNA coding for the enzyme XA799, and corresponding amino acid sequence, are described in Figs. 14-1 to 14-4 (Figs. 16-1 to 16-3 of the above patent application). Therefore, for the present purposes a DNA coding for any enzyme, or derivatives thereof, can be prepared by starting from the above-mentioned cloned cDNAs.

As an example of a native enzyme, a polypeptide having an amino acid sequence from the -37th amino acid to the 363rd amino acid encoded by the cDNA described in Figs. 13-1 to 13-3 can be mentioned, and as an example of a enzyme derivative, a polypeptide having an amino acid sequence from the -39th amino acid to the 692nd amino acid encoded by cDNA described in Figs. 14-1 to 14-3, and an additional C-terminal leucine, can be mentioned. To express the above-mentioned polypeptides, expression plasmids pKDPXA457 and pKDPXA799 BglII were constructed in which a DNA coding for a desired polypeptide is inserted under the control of an SV40 promoter. These plasmids were then used to transfect CHO (Chinese hamster ovary) cells, so as to obtain transfectants CHO/pKDPXA457-α and CHO/pKDPXA799 BglII-α. The transfected cells were sequentially cultured in media having increasing methotrexate concentrations to amplify the transfected gene, and finally, the cells, which were subjected to the amplification, were screened to obtain a clone secreting a large amount of a desired protein, and thus was obtained a clone CHO/10C which secretes a C-terminal α-amidating enzyme in a culture supernatant in an amount of 2000 units/ml. The secreted C-terminal α-amidating enzyme, designated as XA799 BglII, can be selectively precipitated by ammonium sulfate. Accordingly, a novel process for the production of a C-terminal α-amidating enzyme, which process is useful for the industrial production of a large amount of the enzyme, was created.

### (3) Determination of optimum condition for amidating reaction

As described above, although eukaryotic cells contain different types of C-terminal α-amidating enzymes, a detailed substrate specificity and an optimum condition for a reaction have not been clarified.

Usually, C-terminal α-amidating enzymes require a molecular oxygen, copper cation (Cu²⁺), ascorbic acid and catalase to obtain a maximum activity thereof. These essential requirements were determined mainly by using a synthetic substrate such as D-Tyr-Val-Gly.

On the other hand, it was found that some kinds of partially or completely purified C-terminal α-amidating enzymes have particular optimum conditions, including an optimum pH.

Accordingly, where a C-terminal α-amidating enzyme is used in-vitro to convert a precursor of a desired protein to a corresponding amidated protein, an optimum condition for the C-terminal α-amidating enzyme is not the same as optimum condition for the precursor as a substrate for the enzyme. For example, as described above, where a C-terminal α-amidating enzyme partially purified from the pig pituitary gland is used to produce human calcitonin, an optimum condition for the enzyme reaction makes the solubility of a precursor of human calcitonin too low to obtain the human calcitonin.

Accordingly, to efficiently produce a desired amidated protein or peptide, an optimum condition for an in-vitro enzyme reaction, depending on a particular combination of the C-terminal α-amidating enzyme used and the desired protein or peptide, must be determined.

Accordingly, we determined an optimum condition for the case wherein the enzyme XA799 BglII is used to produce human calcitonin from a precursor thereof, i.e., hCT-Gly. The determined condition includes a) a concentration of ascorbic acid, b) a concentration of catalase, c) a concentration of copper cation (Cu²⁺), d) a buffer, and a concentration and pH thereof, and e) a concentration of a precursor, i.e., a substrate. As a result, it was found that a) an optimum concentration of ascorbic acid is 1 to 7 mM, and that at a concentration higher than 20 mM, the efficiency of amidation is lowered; b) the catalase is required in a concentration of at least 1 »g/ml; c) when the copper cation concentration was increased at 0 to 10 »M, efficiency of amidating reaction was increased, and that at a concentration higher than 10 »M, the concentration has no effect on the efficiency of amidating reaction; d) for the type, concentration, and pH of the buffer, 10 mM ammonium acetate at a pH of 6 to 7 is the optimum; and e) a concentration of the substrate of up to 5 mg/ml is acceptable. Note, under the above-mentioned condition, the solubility of the human calcitonin precursor hCT-Gly is sufficient to produce human calcitonin.

Accordingly, on the basis of the above-mentioned condition, human calcitonin can be efficiently produced from the precursor hCT-Gly using the enzyme XA799 BglII.

Using the techniques disclosed herein, a precursor of an amidated peptide or protein represented by the formula R-X-Gly, such as a human calcitonin precursor hCT-Gly, can be efficiently produced from a starting peptide or protein represented by the formula R-X-Gly-B containing a precursor moiety, such as a starting peptide for the human calcitonin precursor hCT-Gly-Lys-Lys-Arg, using carboxypeptidase B (CpB); a C-terminal α-amidating enzyme can be efficiently produced using an eukaryotic host such as animal cells; and, a desired amidated peptide or protein can be efficiently produced using a C-terminal α-amidating enzyme under an optimum condition for an enzyme reaction.

The enzyme activity is assayed by using a reaction wherein a substrate generally represented by R-X-Gly is converted to R-X-NH₂ , for example, a reaction wherein a synthetic substrate [¹²⁵I]-Ac-Tyr-Phe-Gly is converted to [¹²⁵I]-Ac-Tyr-Phe-NH₂.

Namely, a labeled substrate (labeled R-X-Gly) is subjected to a reaction with a test enzyme solution in a Tris-HCl buffer, Tris-HCl buffer and ethyl acetate are added to this reaction mixture, and after mixing, the whole is centrifuged to separate an organic phase and an aqueous phase. Since a major portion of the unreacted labeled substrate (labeled R-X-Gly) retains in an aqueous phase, and an amidated labeled products (labeled R-X-NH₂) transfers to an organic phase, the substrate and the product can be easily separated.

In examples of the present invention, a C-terminal α-amidating enzyme embodying the invention was assayed using a synthetic peptide [¹²⁵I]-Ac-Tyr-Phe-Gly as a substrate, according to the following procedure. [¹²⁵I]-Ac-Tyr-Phe-Gly (1 pmole, 70,000 - 150,000 cpm) was incubated with an enzyme preparation, in a final volume of 250 »l containing a 0.2 M Tris-HCl buffer (pH 7.0), 2 »M CuSO₄ , 0.25 mM ascorbic acid, 25 »g catalase (Boehringer), and 0.1% Lubrol (PX type, Nakarai Chemicals). The reaction mixture was kept at 37°C for 1 to 4 hours, and then 0.75 ml of 1 M Tris-HCl buffer (pH 7.0) and 2 ml of the organic phase of an ethyl acetate/water mixture was added. The reaction mixture thus prepared was mixed vigorously on a Vortex mixer, and after centrifugation at 3000 rpm for 3 mins, the organic phase thus separated was transferred to another test tube. The radioactivity in the organic and aqueous layers was measured by a gamma scintillation counter. Under the conditions described above, over 98% of the radioactivity of the authentic [¹²⁵I]-Ac-Tyr-Phe-Gly was retained in an aqueous phase, and over 98% of the radioactivity of the authentic [¹²⁵I]-Ac-Tyr-Phe-NH₂ was transferred to an organic phase.

The conversion yield is calculated from the ratio of the radioactivity in an organic phase, such as an ethyl acetate phase, to the total radioactivity. In this assay, one unit is defined as the enzyme activity that gives a fifty percent conversion of a 1 pmole substrate, such as [¹²⁵I]-Ac-Try-Phe-Gly, to [¹²⁵I]-Ac-Tyr-Phe-NH₂ for 1 hour.

### EXAMPLES

The present invention will now be further illustrated by, but is no by means limited to, the following examples.

### Example 1. Production of human calcitonin precursor (hCT-Gly)

The starting peptide hCT-Gly-Lys-Lys-Arg is the same compound as the HPCT described in Japanese Patent Application No. 63-49723 (EP 0281418 A2), and a process for the production of this compound is described in the cited application.

First, 280 mg of the peptide hCT-Gly-Lys-Lys-Arg was completely dissolved in 30 ml of 0.1 N acetic acid, and to the solution were added 30 ml of Tris-HCl (pH 8.0) and water to prepare 230 ml of a reaction mixture (pH 7.8). Next, to the reaction mixture was added 560 »g of carboxypeptidase B (Sigma), and a reaction was carried out at 37°C for 30 minutes. To monitor the progress of the reaction, aliquots of the reaction mixture were obtained and subjected to high performance liquid chromatography using a YMC Packed column A-302 (0.46 cm x 15 cm; Murayama Kagaku Kenkyusho), and eluted by a linear gradient formed by 0.1% trifluoroacetic acid (TFA) and 50% CH₃CN in 0.1% TFA to separate a target peptide hCT-Gly, a reaction intermediate hCT-Gly-Lys-Lys and hCT-Gly-Lys, and the starting material hCT-Gly-Lys-Lys-Arg, and as a result, the reaction was completed at 37°C in 30 minutes (see Fig. 1). The hCT-Gly was isolated by applying the reaction mixture to a YMC Packed column D-ODS-5 (2 cm x 25 cm; Murayama Kagaku Kenkyusho) for HPLC, and eluting with 0.1% TFA - 50% CH₃CH. Next, fractions containing the hCT-Gly were combined and lyophilized, to obtain 235 mg of the hCT-Gly. The product was confirmed by hydrolyzing the product in 6 N hydrochloric acid for 24 hours, and analyzing the hydrolyzate by an amino acid analyzer (Hitachi Seisaku Sho 835-20) to obtain an amino acid composition which conforms to a theoretical value. Further, an amino acid sequence of the product was partially determined using a protein sequencer (Applied Biosystems; 470A Protein sequencer), and the obtained sequence conforming to that of hCT-Gly. The result of the amino acid analysis is set forth below; note the numbers in parentheses are theoretical values.
Asx 2.83 (3), Thr 4.51 (5), Ser 0.91 (1),
Glx 1.94 (2), Pro 2.04 (2), Gly 4.83 (5),
Ala 1.78 (2), Val 0.98 (1), Met 0.97 (1),
Ile 0.95 (1), Leu 2.00 (2), Tyr 0.95 (1),
Phe 2.89 (3), Lys 0.99 (1), His 0.97 (1).

### Example 2. Production of C-terminal α-amidating enzyme

### (1) Construction of animal cell expression plasmids pKDPXA457 and pKDPXA799 BglII

The plasmids pKDPXA457 and pKDPXA799 BglII contain a cDNA coding for a protein having an amino acid sequence from the -37th amino acid to the 363th amino acid shown in Figs. 13-1 to 13-3, and a cDNA coding for a protein having an amino acid sequence from the -39th amino acid to the 692th amino acid in Figs. 14-1 to 14-3 and an additional C-terminal amino acid Leu, respectively. The cDNA is positioned downstream of SV40 promoter, and is followed by poly A addition signal derived from rabbit β-globin gene. Due to this construction, the cDNA is transcribed under the control of the SV40 promoter, the resulting mRNA is spliced, and poly A is added to the spliced mRNA. Further, the expression plasmid contains a dhfr (dihydrofolate reductase) gene under the control of an SV40 early promoter, whereby the inserted gene is amplified in animal cells.

Note, E. coli transformed with the plasmid pKDPXA457 was designated as E. coli SBM -300 and deposited with the Fermentation Research Institute Agency of Industrial Science and Technology (FRI), 1-3, Higashi 1-chome, Tsukuba-shi, Ibaraki-ken 305, Japan, as FERM BP-2235 under the Budapest Treaty, on January 11, 1989; and E. coli transformed with the plasmid pKDPXA799 BglII was designated as E. coli SBM 301 and deposited with the FRI as FERM BP-2236 under the Budapest Treaty, on January 11, 1989.

### (2) Transfection of pKDPXA457 or pKDPXA799 BglII into CHO cells

The plasmid pKDPXA457 or pKDPXA799 BglII was transfected in CHO cells lacking the dhfr (dihydrofolate reductase) gene, by a calcium phosphate coprecipitation method. Note, the CHO cell line lacking the dhfr gene was designated as CHO dhfr⁻ Cell SBM 306, and deposited with the FRI as FERM BP-2241 under the Budapest Treaty, on January 11, 1989.

First, CHO dhfr⁻ cells were cultured in a Minimum Essential Medium (MEM) Alpha Medium (GIBCO, α⁺ MEM) containing nucleic acid supplemented with 10% fetal bovine serum (FBS) (Flow. Lab.), and 50 U/mℓ penicillin G and 50 »g/ml streptomycin. Then, 12 hours before the transfection, the cultured cells were plated in an 80 cm² T-flask (T80; Nunc) at a density of 1.6 x 10⁶ cells/30 ml/T80 flask, and 4 hours before the transfection, the medium was replaced by 30 ml of fresh α⁺ MEM supplemented with a 10% FBS and the antibiotics.

On the other hand, 10 »g of the plasmid pKDPXA457 or pKDPXA799 BglII was dissolved in 240 »l of sterilized water, and to the solution was added the same volume of a Buffer A (0.5 M CaCl₂ , 0.1 M HEPES), and the whole was mixed. After allowing to stand at a room temperature for 10 minutes, 480 »l of a Buffer B (0.28 M NaCl, 0.05 M HEPES, 0.75 mM NaH₂PO₄ , 0.75 mM Na₂HPO₄) was added to the mixture, and the mixture was mixed on a Vortex mixer for several seconds. The mixture was then allowed to stand at a room temperature for 20 to 30 minutes, to form a calcium phosphate gel containing the plasmid.

Next, 960 »l of the calcium phosphate gel containing the plasmid was added to the above-mentioned CHO dhfr⁻ cells (1.6 x 10⁶ cells/30 ml/T80 flask), and the flask was kept for four hours. Then, the cells were washed once with 10 ml of α⁺ MEM not containing FBS, and to the cells was added 5 ml/T80 flask of a mixture of α⁺ MEM/glycerol (4:1) containing 10% FBS. After exactly one minute, the medium was removed by aspiration, and 30 ml of α⁺ MEM containing 10% FBS was added to the cells, which were then kept at 37°C under the presence of 5% CO₂. After cultivation for 4 days, cells were dispersed with a 0.25% trypsin solution (Chiba Kessei), and inoculated in a nucleic acid-free MEM medium (α⁻ MEM) supplemented with a 10% dialyzed fetal bovine serum (FBS^{d}, HAZELTON) at a density of 1.6 x 10⁶ cells/30 ml/T80 flask. The cells were cultured for 10 days, and cells which survived this medium were selected as transfected cells. The cells infected with pKDPXA457 were designated as CHO/pKDPXA457-α, and the cells infected with pKDPXA799 BglII were designated as CHO/pKDPXA799 BglII-α, and were used for the following experiments.

### (3) Amplification of gene

To amplify the genes (pKDPXA457 or pKDPXA799 BglII) in the cells CHO/pKDPXA457-α and CHO/pKDPXA799 BglII-α, respectively, the cells were sequentially cultured in media containing an increasing concentrations, i.e., 30 nM, 100 nM, 300 nM, and 1000 nM, of methotrexate (MTX) (Sigma) while selecting an MTX resistant cell at each step. Next, cells which had acquired resistance to 1000 nM MTX, designated as CHO/pKDPXA457-1 and CHO/pKDPXA799 BglII-1, respectively, were inoculated to a fresh medium at a density of 1.6 x 10⁶ cells/30 ml/T80 flask, and cultured at 37°C in the presence of 5% CO₂ for 4 days. Then, a portion of the culture supernatant was used to determine the C-terminal α-amidating enzyme activity, using a synthetic substrate (¹²⁵I]-Ac-Tyr-Phe-Gly. As a result, culture supernatants of CHO/pKDPXA457-1 and CHO/pKDPXA799 BglII-1 exhibited an activity of 1 unit/ml and 310 units/ml, respectively.

### (4) Establishment of clones having great ability to produce C-terminal α-amidating enzyme

Since the above-mentioned experiment showed that cells transfected with the pKDPXA799 BglII exhibited an enzyme activity higher than that provided by cells transfected with pKDPXA457, to establish a clone having a higher enzyme productivity, the MTX 100 nM-resistant cells CHO/pKDPXA799 BglII was subjected to a limiting dilution cloning method. Namely, CHO/pKDPXA799 BglII cells were distributed to wells of a plate so in such a manner that each well contained an average of 3, 1.5, 0.75 or 0.375 cells, and the cells were cultured in 100 »l/well α⁻ MEM for one week. After the culture, 30 wells in which a development of a single colony was microscopically observed were selected, 100 »l of α-MEM was added to the wells, and a further cultivation was continued for one week. These supernatants from the 30 wells were assayed to determine the enzyme activity thereof, and it was found that a clone designated as CHO/9C exhibited the highest enzyme activity at 910 units/ml.

Next, the clone CHO/9C was sequentially cultured in media containing increasing concentrations, i.e., 0.1, 0.3, 1, 3, 10 and 30 »M, of MTX, while obtaining MTX-resistant clones at each stage to obtain cell clones having a higher resistance to MTX. The MTX-resistant cells thus obtained were cultured for 4 days at a density of 1.6 x 10⁶ cells/30 ml/T80 flask, the supernatants were assayed to determine the enzyme activity thereof, and it was found that an MTX 3 »M-resistant cells exhibited the highest enzyme activity at 2860 units/ml. To establish a clone having a higher enzyme productivity, the MTX 3 »M-resistant 9C cells were further subjected to a cloning procedure as described above, and as a result, a clone exhibiting a more than 2000 units/ml C-terminal α-amidating enzyme activity was established, and was designated as CHO/10C.

### (5) Cultivation of cell clone CHO/10C cells

The cell clone CHO/10C established in Example 2(4) was cultured to produce a C-terminal α-amidating enzyme derivative. Namely, 1 x 10⁷ CHO/10C cells and 500 ml of α⁻ MEM were introduced to a 1850 cm² roller bottle (Falcon), and cultivation was carried out at 37°C for one week. Next, the cells were sequentially cultured in α⁻ MEM containing 10% FBS, α⁻ MEM containing 3% FBS, and in α⁻ MEM containing 1% FBS at 37°C for 24 hours. A supernatant was recovered from the culture containing 1% FBS, and used for the following experiment. Note, this supernatant exhibited an enzyme activity of about 4000 units/ml of enzyme activity, and the enzyme was designated as XA799 BglII.

### (6) Purification of enzyme XA799 BglII

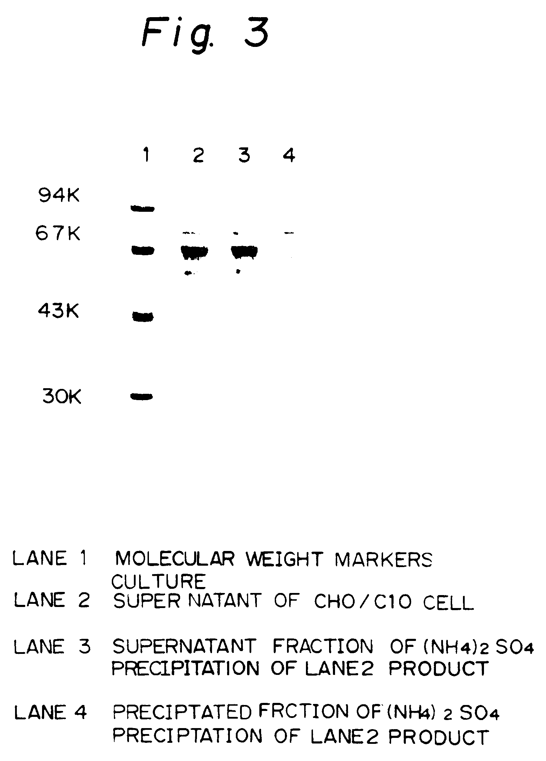

To 1 ℓ of the supernatant obtained in Example 3(5), was added ammonium sulfate to a final concentration of 45%, and the resulting precipitate was collected by centrifugation and redissolved in 20 ml of 50 mM Tris-HCl buffer (pH 7.0). Next, the enzyme fraction thus obtained was assayed to determine the enzyme activity thereof, using a synthetic substrate, and it was found that most of the enzyme activity was recovered in the precipitated fraction. Further, an SDS-PAGE analysis of each fraction showed that most of the proteins (mainly BSA) derived from medium were eliminated from the precipitated fraction, as can be seen in Fig. 3.

The XA799 BglII thus obtained was used to study the condition required for an in-vitro amidation, in the following experiments.

### Example 3. Study of condition for in-vitro amidation

To study the condition required for an in-vitro amidation, the hCT-Gly obtained in Example 1 and the XA799 BglII purified in Example 2(6) were used. In the reaction, 1 ml of reaction mixture was incubated at 37°C, and 50 »l of samples were obtained at 0.5, 1, 2 and 4 hours of the reaction. Then to the samples was added 950 »l of 5 N acetic acid, and 20 »l of the mixture was subjected to C8-HPLC, using a column YMC A-302 (4.6 x 150 mm) and eluting with a gradient of 24% to 45% acetonitryl in 10% ammonium acetate for 18 minutes, to measure the resulting hCT and residual hCT-Gly. Note, as reaction conditions (1) an ascorbic acid concentration, (2) a catalase concentration, (3) a copper cation (Cu²⁺) concentration, (4) a pH of the buffer, and (5) a buffer concentration and the hCT-Gly concentration, were studied, taking into consideration the corresponding conditions obtained for the conversion of a synthetic substrate [¹²⁵I]-Ac-Tyr-Phe-Gly to [¹²⁵I]-Ac-Tyr-Phe-NH₂ (see Japanese Patent Application No. 62-306867; EP 0299790 A2).

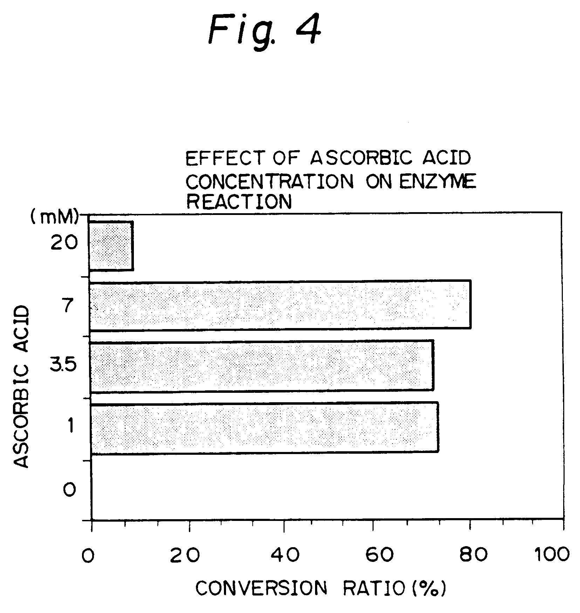

### (1) Ascorbic acid concentration (Fig. 4)

The effect of the ascorbic acid concentration on the amidating reaction was tested at 0, 1.0, 3.5, 7, and 20 mM of ascorbic acid. The remaining components were as follows:
2.5 mg/ml hCT-Gly
100 mM Tris-HCl (pH 7.0)
25 »g/ml Catalase
70 »M CuSO₄
1860 U/ml XA799 BglII.

The conversion ratio [100 x hCT/(hCT + hCT-Gly)] at one hour of the reaction is shown in Fig. 4. The reaction does not proceed in the absence of ascorbic acid, but proceeds satisfactorily at 1 to 7 mM of ascorbic acid. At 20 mM of ascorbic acid, however, the conversion ratio was decreased.

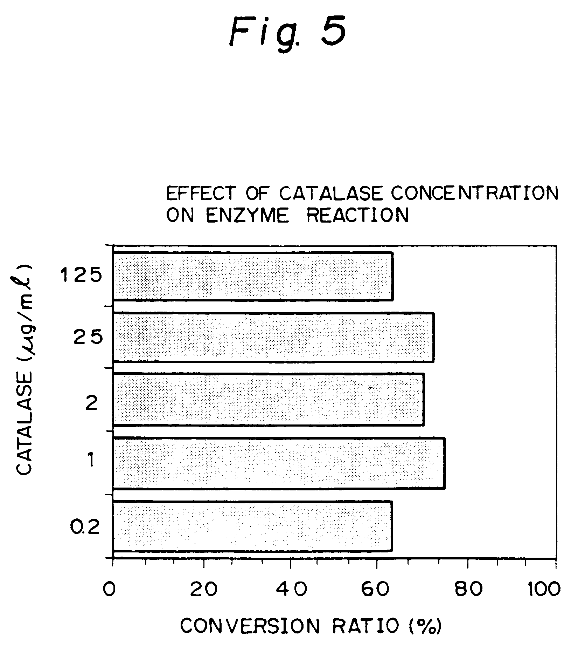

### (2) Catalase concentration (Fig. 5)

The effect of the catalase concentration on the amidating reaction at 0.2, 1, 2, 25, and 125 »g/ml catalase was tested. The remaining components were as follows:
2.5 mg/ml hCT-Gly
100 mM Tris-HCl (pH 7.0)
3.5 mM Ascorbic acid
70 »M CuSO₄
1500 U/ml XA799 BglII.

The conversion ratio at one hour of the reaction was 60 to 75%, and did not differ according to the catalase concentration (Fig. 5), but at 1 »g/ml or more of the catarase concentration conversion ratio reached 100% in 2 hours, and at 0.2 »g/ml catalase, the conversion reached only 93% in 4 hours.

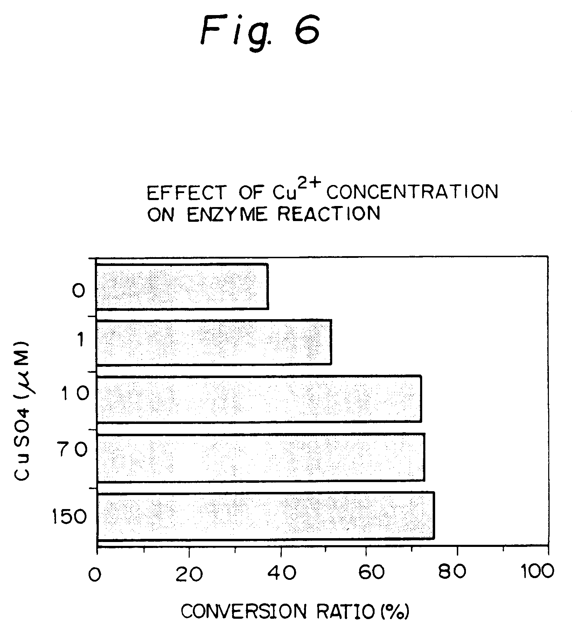

### (3) CuSO₄ (Cu²⁺ cation) concentration (Fig. 6)

The effect of CuSO₄ concentration on the amidating reaction was tested at 0, 1, 10, 70 and 150 »M CuSO₄. The remaining components were as follows:
2.5 mg/ml hCT-Gly
100 mM Tris-HCl (pH 7.0)
25 »g/ml Catalase
3.5 mM Ascorbic acid
1500 U/ml XA799 BglII.

The conversion ratio [100 x hCT/(hCT + hCT-Gly)] at one hour of the reaction is shown in Fig. 6. The amidating reaction increased as the CuSO₄ concentration increased from 1 to 10 »M, but a further increase of the CuSO₄ concentration did not improve the amidation ratio.

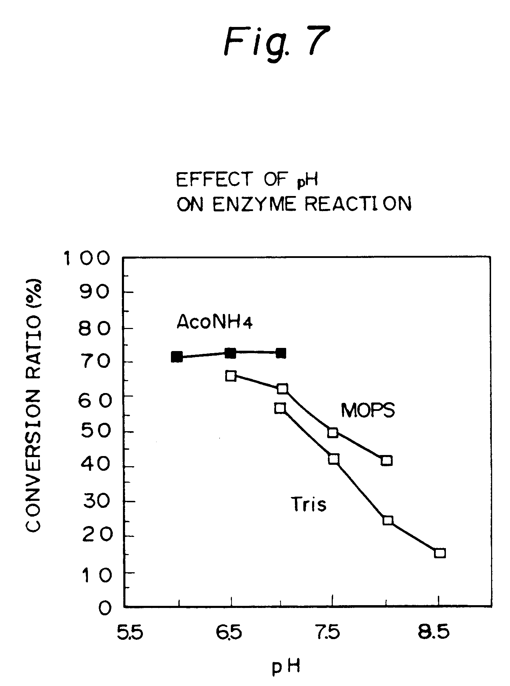

### (4) Buffer (pH) (Fig. 7)

The amidating reaction was tested for 100 mM Tris-HCl (pH 7.0, 7.5, 8.0, 8.5), 100 mM MOPS (pH 6.5, 7.0, 7.5 and 8.0), and 100 mM of ammonium acetate (pH 6.0, 6.5 and 7.0). The remaining components in the reaction mixture were as follows:
2.5 mg/ml hCT-Gly
20 »g/ml Catalase
2.0 mM Ascorbic acid
10 »M CuSO₄
1000 U/ml XA799 BglII.

The conversion ratio [100 x hCT/(hCT + hCT-Gly)] at one hour of the reaction is shown in Fig. 7. The optimal pH was 6 to 7, and in ammonium acetate, the reaction rate was higher than that in MOPS.

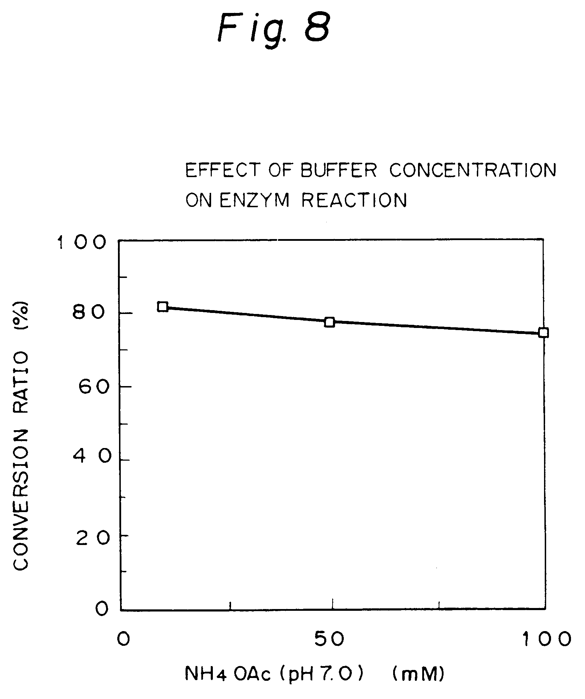

### (5) Buffer (concentration) (Fig. 8)

The effect of ammonium acetate on the amidating reaction was tested at 10, 50 and 100 mM of ammonium acetate. The remaining components in the reaction mixture were as follows:
2.5 mg/ml hCT-Gly
20 »g/ml Catalase
10 »M CuSO₄
2.0 mM Ascorbic acid
1000 U/ml XA799 BglII.

The conversion ratio [(100 x hCT/(hCT + hCT-Gly)] at one hour of the reaction is shown in Fig. 8. The conversion ratio was about 80%, and substantially did not differ in accordance with the ammonium acetate concentration, but at a lower ammonium acetate concentration, the conversion ratio was slightly higher.

### (6) Substrate (hCT-Gly) concentration

The effect of the hCT-Gly concentration on the amidating reaction was tested at 2.5 and 5 mg/ml hCT-Gly. The remaining components in the reaction mixture were as follows:
10 mM Ammonium acetate (pH 6.0)
20 »g/ml Catalase
10 »M CuSO₄
2.0 mM Ascorbic acid
1000 U/ml XA799 BglII.

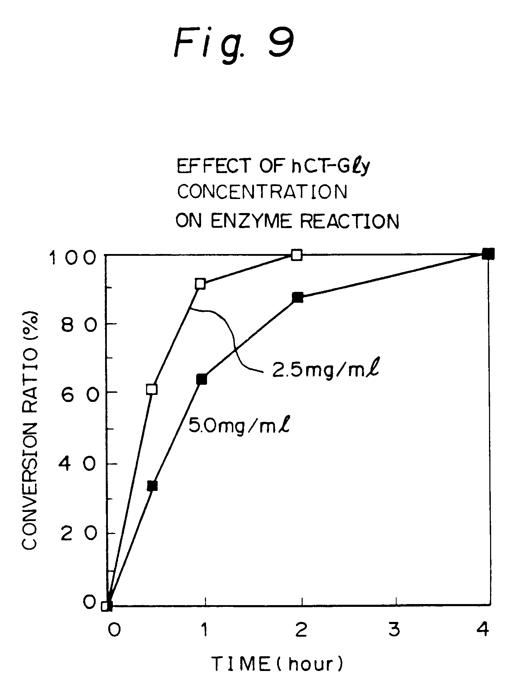

The relationship between the reaction time and the conversion ratio [(100 x hCT/(hCT + hCT - Gly)] for each hCT-Gly concentration is shown in Fig. 9. At a 2.5 mg/ml substrate, the amidation was completed in 2 hours, and at a 5.0 mg/ml substrate, the amidation was completed in 4 hours. Since the XA799 BglII concentration was the same, an amount of XA799 BglII per hCT-Gly at 5 mg/ml hCT-Gly is a half of that at 2.5 mg/ml hCT-Gly.

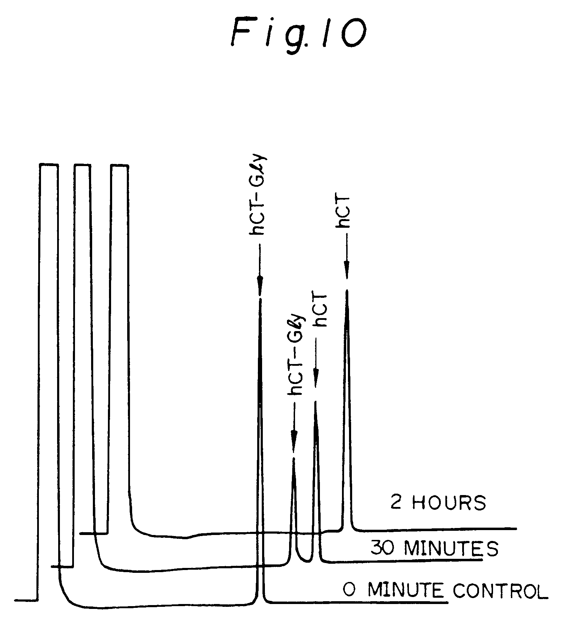

### Example 4. Production of hCT (Fig. 10)

An amidating reaction was carried out to produce hCT, under the following condition:
2.5 mg/ml hCT-Gly
10 mM Ammonium acetate (pH 6.0)
20 »g/ml Catalase
10 »M CuSO₄
2.0 mM Ascorbic acid
1000 U/ml XA799 BglII.

Samples were obtained prior to starting the reaction, 0.5 hours after starting the reaction, and 2 hours after starting the reaction, and subjected to C18-HPLC. Charts of the elution are shown in Fig. 10. As time elapsed, a peak representing the substrate hCT-Gly (retention time 11.8 minutes) was reduced and a peak representing the product hCT (retention time 12.9 minutes) was increased. After a reaction for 2 hours, the reaction mixture was subjected to C18-HPLC, and the hCT was isolated and purified. The hCT was obtained in a yield of 93.5%, as determined by an amino acid analysis of the hCT.

### Example 5. Confirmation of hCT

The Human calcitonin (hCT) produced in Example 4 was confirmed as follows.
1) The retention time on HPLC under the condition described in Example 3 for hCT prepared in Example 4 was exactly the same as that of synthetic human calcitonin obtained from Peptide Institute, Osaka, Japan.
2) The amino acid composition of the hCT prepared in Example 4, obtained by amino acid analysis, conformed to the theoretical values shown in the parentheses.
   Asx 2.99 (3), Thr 4.72 (5), Ser 0.91 (1),
   Glx 2.00 (2), Pro 1.97 (2), Gly 3.96 (4),
   Ala 1.97 (2), Val 1.00 (1), Met 0.98 (1),
   Ile 0.97 (1), Leu 2.00 (2), Tyr 1.02 (1),
   Phe 2.95 (3), Lys 1.00 (1), His 0.97 (1).
3) The entire amino acid sequence, except for the C-terminal Pro-NH₂, of the product in Example 4 was confirmed to be the same as that of human calcitonin.
4) To confirm the C-terminal Pro-NH₂ structure of the hCT produced in Example 4, the hCT was treated with lysyl endoprotease (Wako Junyaku Kogyo, Japan) to obtain a C-terminal fragment hCT[19-32]. Note, since hCT has a lysine residue at the 18 position, the lysyl endoprotease cleaves the hCT between the 18th amino acid and 19th amino acid. The molecular weight of the fragment hCT[19-32] was determined by fast bombardment (FAB) mass spectrometry, and as a result, it was found that the molecular weight was the same as the theoretical molecular weight, thereby showing the presence of C-terminal Pro-NH₂.

From the above result, it was confirmed that the product obtained in Example 4 was human calcitonin.

### Example 6. Effect of C-terminal amino acids on production of fused protein βgal197 SHPCT

To study an effect of C-terminal amino acids on the production of a fused protein βgal97HPCT, the productivity of the following fused proteins, wherein the βgal197S(LE)[GKKR] used in Example 1 has been modified at the C-terminus thereof, was determined:
βgal97S(LE)HPCT[GRRR]
βgal97S(LE)HPCT[GR]
βgal97S(LE)HPCT[G].

Note, in the above formula, [LE] means that βgal197S and HPCT is linked via Leu-Glu; and [GRRR], [GR] and [G] mean that the fused proteins have added amino acids Gly-Arg-Arg-Arg, Gly-Arg, and Gly respectively.

Plasmids containing genes coding for the respective above-mentioned proteins were constructed, used to transform E. coli, and the transformants were tested to determine the productivity thereof of the fused protein.

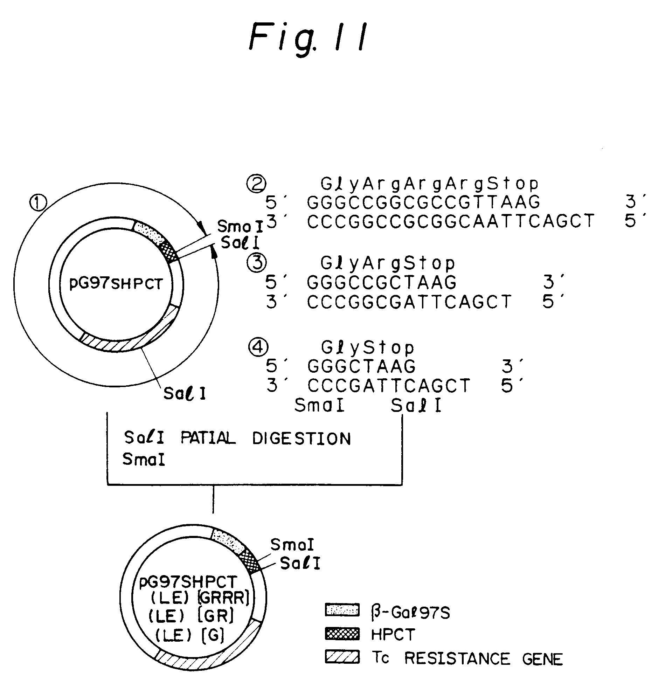

### (1) Construction of plasmids having gene coding for chimeric protein (Fig. 11)

A plasmid pG97SHPCT (Japanese Patent Application No. 63-49723; EP 2081418 A2 Example 8, Fig. 19) was partially digested with SalI, and a 3.9 kb DNA fragment was obtained by agarose gel electrophoresis. Next, this DNA fragment was completely digested with SmaI, and a 3.8 kb DNA fragment (1) was obtained by agarose gel electrophoresis. The DNA fragment (1) was ligated with three pairs of synthetic linkers each having SmaI and SalI ends: 
SmaI SalI
(2) 5′ GGGCCGGCGCCGTTAAG 3′
3′ CCCGGCCGCGGCAATTCAGCT 5′
(3) 5′ GGGCCGCTAAG 3′
3′ CCCGGCGATTCAGCT 5′
(4) 5′ GGGCTAAG 3′
3′ CCCGATTCAGCT 5′
to obtain plasmids pG97SHPCT(LE)[GRRR], pG97HPCT(LE)[GR], and pG97SHPCT(LE)[G], respectively. These plasmids were used to transform E. coli W3110, to obtain transformants W3110/pG97SHPCT(LE)[GRRR], W3110/pG97SHPCT(LE)[GR], and W3110/pG97SHPCT(LE)[G].

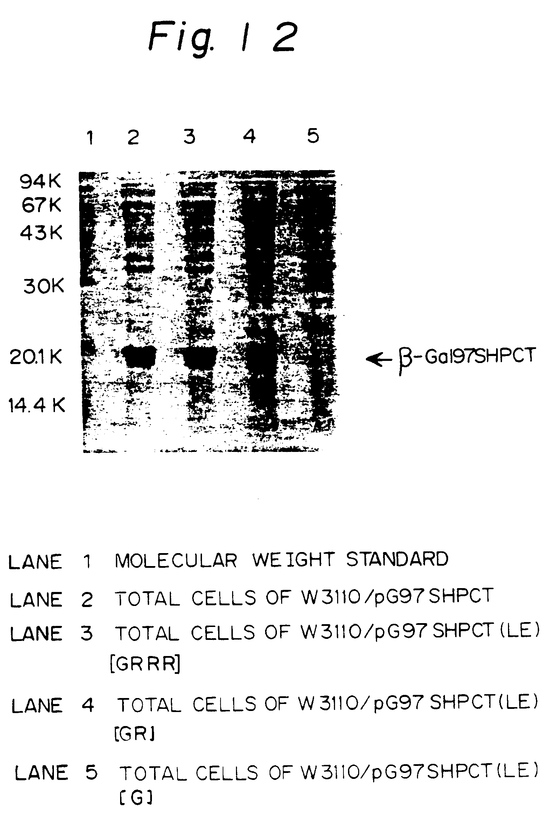

### (2) Productivity test (Fig. 12)

The transformants W3110/pG97SHPCT(LE)[GRRR], W3110/pG97SHPCT(LE)[GR] and W3110/pG97SHPCT(LE)[G], thus prepared, and a control W3110/pG97SHPCT, were separately cultured in a medium (2.4% trypton, 1.2% yeast extract, and 0.5% glycerol) supplemented with tetracycline for 16 hours, and each culture was subjected to SDS-plyacrylamide gel electrophoresis. As shown in Fig. 12, the productivity of the fused protein is clearly higher for fused proteins having C-terminal basic amino acid(s) than for fused protein not having C-terminal basic amino acid(s).

Reference under Rule 13-2 under the Budapest treaty to deposited microoganisms:
Depositor Institute: Fermentation Research Institute, Agency of Industrial Science and Technology;
Address: 1-3, Tsukuba-shi 1-chrome, Ibaraki-ken, 301 Japan;
Identification of deposited microorganisms, deposition numbers and deposition date;
1. E. coli SBM 300 FERM BP-2235 January 11, 1989.
2. E. coli SBM 301 FERM BP-2236 January 11, 1989.
3. CHO dhfr(-) Cell SBM 306 FERM BP-2241 January 11, 1989.

## Claims (Claims for the following Contracting State(s): AT, BE, CH, DE, ES, FR, GB, IT, LI, LU, NL, SE)

1. A process for production of a peptide or protein represented by a formula (II):
R-X-Gly (II)
wherein X represents any amino acid residue, Gly represents a C-terminal glycine residue, and R represents a remaining portion of the peptide or protein, comprising a step of:
hydrolyzing a peptide or protein represented by a formula (I):
R-X-Gly-B
wherein X represents any amino acid residue, Gly represents a glycine residue, B represents a lysine or arginine residue, or an oligopeptide consisting essentially of lysine or arginine residues or a combination of lysine and arginine residues, with carboxypeptidase B.

2. A process according to claim 1, wherein the peptide or protein represented by the formula (II) is a precursor of human calcitonin.

3. A process for the production of human calcitonin, represented by the formula
R-NH₂
in which the -NH₂ group is of the α-amidated C-terminal amino acid of the calcitonin molecule, comprising treating a peptide or protein of the formula
R-Gly
in which Gly represents a C-terminal glycine residue with a C-terminal α-amidating enzyme of human thyroid gland origin or a derivative thereof.

4. A process for production of a peptide or protein represented by a formula (III):
R-X-NH₂ (III)
wherein X-NH₂ represents a C-terminal α-amidated amino acid and R represents a remaining portion of the peptide or protein, comprising steps of:
(1) hydrolyzing a peptide or protein represented by the formula (I):
R-X-Gly-B (I)
wherein X represents any amino acid residue, Gly represents a glycine residue, B represents a lysine or arginine residue, or an oligopeptide consisting essentially of lysine or arginine residues or a combination of lysine and arginine residues, and R represents a remaining portion of the peptide or protein residues, with carboxypeptidase B, to produce an intermediate peptide or protein represented by the formula (II):
R-X-Gly (II)
wherein X represents any amino acid residue to be α-amidated, Gly represents a C-terminal glycine residue, and R represents a remaining portion of the peptide and protein; and
(2) treating the intermediate peptide or protein represented by the formula (II) with a C-terminal α-amidating enzyme or a derivative thereof.

5. A process according to claim 4, wherein the peptide or protein represented by the formula (III) is human calcitonin.

6. A process according to claim 4, wherein the C-terminal α-amidating enzyme is of Xenopus laevis or human thyroid origin.

7. A process for production of a C-terminal α-amidating enzyme or a derivative thereof, comprising the steps of
culturing animal cells transfected with a plasmid containing a DNA coding for said enzyme or a derivative thereof and capable of expressing same; and
recovering the enzyme or a derivative thereof.

8. A process according to claim 7, wherein the C-terminal α-amidating enzyme is XA457 having an amino acid sequence starting with the -37th amino acid and terminating with the 363rd amino acid represented n Figs. 13-1 to 13-3.

9. A process according to claim 7, wherein the derivative of the C-terminal α-amidating enzyme is XA799 BglII having an amino acid sequence starting from the -39th amino acid and terminating at the 692nd amino acid represented in Figs. 14-1 to 14-3 and C-terminal leucine.

10. A process according to claim 7, wherein the transfected animal cells are CHO dhfr(-) cells transfected with a plasmid comprising an SV40 promoter, a DNA coding for an amino acid sequence starting with the -39th amino acid and terminating with the 692nd amino acid represented in Figs. 14-1 to 14-3 and C-terminal leucine, with the DNA being operably linked with the SV40 promoter, and further comprising a dhfr gene, and the transfected animal cells are subjected to gene amplification by a sequential culturing in media containing methatorexate in concentrations increasing up to 3 »M, to produce the enzyme derivative in an amount of 2000 unit/ml supernatant.

11. A process for production of a peptide or protein represented by the formula (II):
R-X-Gly (II)
wherein X represents any amino acid residue, Gly represents a C-terminal glycine residue, and R represents a remaining portion of the peptide or protein, comprising the steps of:
(1) obtaining a fused protein represented by the formula (0):
P-R-X-Gly-B (0)
wherein P represents a partner protein of the fused protein, X represents any amino acid residue, Gly represents a glycine residue, B represents a C-terminal lysine or arginine residue or an oligopeptide consisting essentially of lysine or arginine residues or a combination of lysine and arginine residues, and R represents a remaining portion of the protein, by culturing a host transformed with an expression vector containing a DNA coding for the fused protein;
(2) recovering the fused protein;
(3) cleaving the fused protein by a conventional method to form a partner protein (P) and a peptide or protein represented by the formula (I):
R-X-Gly-B (I)
wherein R, X, Gly and B have the same meanings as defined under the formula (0);
(4) separating the peptide or protein (I) from the partner protein (P) on the basis of a difference of the isoelectric points thereof; and
(5) hydrolysing the peptide or protein (I) with carboxypeptidase B to form the desired peptide or protein (II).

12. A process according to claim 11, wherein the peptide or protein represented by the formula (II) is a precursor of human calcitonin.

13. A process for production of a peptide or protein represented by the formula (III):
R-X-NH₂ (III)
wherein X-NH₂ represents a C-terminal α-amidated amino acid, and R represents a remaining portion of the peptide or protein, comprising the steps of:
(1) obtaining a fused protein represented by the formula (0):
P-R-X-Gly-B (0)
wherein P represents a partner protein of the fused protein, X represents any amino acid residue, Gly represents a glycine residue, B represents a C-terminal lysine or arginine residue, or an oligopeptide consisting essentially of lysine or arginine residues or a combination of lysine and arginine residues, and R represents a remaining portion of the protein, by culturing a host transformed with an expression vector containing a DNA coding for the fused protein;
(2) recovering the fused protein;
(3) cleaving the fused protein by a conventional method to form a partner protein (P) and a peptide or protein represented by the formula (I):
R-X-Gly-B (I)
wherein R, X, Gly and B have the same meanings as defined under the formula (0);
(4) separating the peptide or protein (I) from the partner protein (P) on the basis of a difference of the isoelectric points thereof;
(5) hydrolyzing the peptide or protein (I) with carboxypeptidase B to form an intermediate peptide or protein represented by the formula (II):
R-X-Gly (II)
wherein X represents any amino acid residue, Gly represents a C-terminal glycine residue, and R represents a remaining portion of the peptide or protein; and
(6) treating the peptide or protein (II) with a C-terminal α-amidating enzyme or a derivative thereof.

14. A process according to claim 13, wherein the peptide or protein represented by the formula (III) is human calcitonin.

## Claims (Claims for the following Contracting State(s): DK)

1. A process for production of a peptide or protein represented by a formula (II):
R-X-Gly (II)
wherein X represents any amino acid residue, Gly represents a C-terminal glycine residue, and R represents a remaining portion of the peptide or protein, comprising a step of:
hydrolyzing a peptide or protein represented by a formula (I):
R-X-Gly-B
wherein X represents any amino acid residue, Gly represents a glycine residue, B represents a lysine or arginine residue, or an oligopeptide consisting essentially of lysine or arginine residues or a combination of lysine and arginine residues, with carboxypeptidase B.

2. A process according to claim 1, wherein the peptide or protein represented by the formula (II) is a precursor of human calcitonin.

3. A process for the production of human calcitonin, represented by the formula
R-NH₂
in which the -NH₂ group is of the α-amidated C-terminal amino acid of the calcitonin molecule, comprising treating a peptide or protein of the formula
R-Gly
in which Gly represents a C-terminal glycine residue with a C-terminal α-amidating enzyme of human thyroid gland origin or a derivative thereof.

4. A process for production of a peptide or protein represented by a formula (III):
R-X-NH₂ (III)
wherein X-NH₂ represents a C-terminal α-amidated amino acid and R represents a remaining portion of the peptide or protein, comprising steps of:
(1) hydrolyzing a peptide or protein represented by the formula (I):
R-X-Gly-B (I)
wherein X represents any amino acid residue, Gly represents a glycine residue, B represents a lysine or arginine residue, or an oligopeptide consisting essentially of lysine or arginine residues or a combination of lysine and arginine residues, and R represents a remaining portion of the peptide or protein residues, with carboxypeptidase B, to produce an intermediate peptide or protein represented by the formula (II):
R-X-Gly (II)
wherein X represents any amino acid residue to be α-amidated, Gly represents a C-terminal glycine residue, and R represents a remaining portion of the peptide and protein; and
(2) treating the intermediate peptide or protein represented by the formula (II) with a C-terminal α-amidating enzyme or a derivative thereof.

5. A process according to claim 4, wherein the peptide or protein represented by the formula (III) is human calcitonin.

6. A process according to claim 4, wherein the C-terminal α-amidating enzyme is of Xenopus laevis or human thyroid origin.

7. A process for production of a C-terminal α-amidating enzyme or a derivative thereof, comprising the steps of
culturing animal cells transfected with a plasmid containing a DNA coding for said enzyme or a derivative thereof and capable of expressing same; and
recovering the enzyme or a derivative thereof.

8. A process according to claim 7, wherein the C-terminal α-amidating enzyme is XA457 having an amino acid sequence starting with the -37th amino acid and terminating with the 363rd amino acid represented n Figs. 13-1 to 13-3.

9. A process according to claim 7, wherein the derivative of the C-terminal α-amidating enzyme is XA799 BglII having an amino acid sequence starting from the -39th amino acid and terminating at the 692nd amino acid represented in Figs. 14-1 to 14-3 and C-terminal leucine.

10. A process according to claim 7, wherein the transfected animal cells are CHO dhfr(-) cells transfected with a plasmid comprising an SV40 promoter, a DNA coding for an amino acid sequence starting with the -39th amino acid and terminating with the 692nd amino acid represented in Figs. 14-1 to 14-3 and C-terminal leucine, with the DNA being operably linked with the SV40 promoter, and further comprising a dhfr gene, and the transfected animal cells are subjected to gene amplification by a sequential culturing in media containing methatorexate in concentrations increasing up to 3 »M, to produce the enzyme derivative in an amount of 2000 unit/ml supernatant.

11. A process for production of a peptide or protein represented by the formula (II):
R-X-Gly (II)
wherein X represents any amino acid residue, Gly represents a C-terminal glycine residue, and R represents a remaining portion of the peptide or protein, comprising the steps of:
(1) obtaining a fused protein represented by the formula (0):
P-R-X-Gly-B (0)
wherein P represents a partner protein of the fused protein, X represents any amino acid residue, Gly represents a glycine residue, B represents a C-terminal lysine or arginine residue or an oligopeptide consisting essentially of lysine or arginine residues or a combination of lysine and arginine residues, and R represents a remaining portion of the protein, by culturing a host transformed with an expression vector containing a DNA coding for the fused protein;
(2) recovering the fused protein;
(3) cleaving the fused protein by a conventional method to form a partner protein (P) and a peptide or protein represented by the formula (I):
R-X-Gly-B (I)
wherein R, X, Gly and B have the same meanings as defined under the formula (0);
(4) separating the peptide or protein (I) from the partner protein (P) on the basis of a difference of the isoelectric points thereof; and
(5) hydrolyzing the peptide or protein (I) with carboxypeptidase B to form the desired peptide or protein (II).

12. A process according to claim 11, wherein the peptide or protein represented by the formula (II) is a precursor of human calcitonin.

13. A process for production of a peptide or protein represented by the formula (III):
R-X-NH₂ (III)
wherein X-NH₂ represents a C-terminal α-amidated amino acid, and R represents a remaining portion of the peptide or protein, comprising the steps of:
(1) obtaining a fused protein represented by the formula (0):
P-R-X-Gly-B (0)
wherein P represents a partner protein of the fused protein, X represents any amino acid residue, Gly represents a glycine residue, B represents a C-terminal lysine or arginine residue, or an oligopeptide consisting essentially of lysine or arginine residues or a combination of lysine and arginine residues, and R represents a remaining portion of the protein, by culturing a host transformed with an expression vector containing a DNA coding for the fused protein;
(2) recovering the fused protein;
(3) cleaving the fused protein by a conventional method to form a partner protein (P) and a peptide or protein represented by the formula (I):
R-X-Gly-B (I)
wherein R, X, Gly and B have the same meanings as defined under the formula (0);
(4) separating the peptide or protein (I) from the partner protein (P) on the basis of a difference of the isoelectric points thereof;
(5) hydrolyzing the peptide or protein (I) with carboxypeptidase B to form an intermediate peptide or protein represented by the formula (II):
R-X-Gly (II)
wherein X represents any amino acid residue, Gly represents a C-terminal glycine residues and R represents a remaining portion of the peptide or protein; and
(6) treating the peptide or protein (II) with a C-terminal α-amidating enzyme or a derivative thereof.

14. A process according to claim 13, wherein the peptide or protein represented by the formula (III) is human calcitonin.

15. A C-terminal α-amidating enzyme obtainable by the process of claim 7, having an amino acid sequence starting with the -37th amino acid and terminating at the 363rd amino acid shown in Figs. 13-1 to 13-3.

16. A C-terminal α-amidating enzyme derivative obtainable by the process of claim 7 and having an amino acid sequence starting with the -39th amino acid and terminating at the 692nd amino acid shown in Figs. 14-1 to 14-3.

## Patentansprüche (Patentansprüche für folgende(n) Vertragsstaat(en): AT, BE, CH, DE, ES, FR, GB, IT, LI, LU, NL, SE)

1. Verfahren zur Herstellung eines Peptids oder Proteins dargestellt durch die Formel (II):
R-X-Gly (II)
worin X einen beliebigen Aminosäurerest darstellt, Gly einen C-Terminus Glycinrest darstellt und R einen restlichen Teil des Peptids oder Proteins darstellt, das die Schritte umfaßt:
Hydrolyse eines Peptids oder Proteins, dargestellt durch die Formel (I):
R-X-Gly-B
worin X einen beliebigen Aminosäurerest, Gly einen Glycinrest, B einen Lysin- oder Argininrest oder ein Oligopeptid, das im wesentlichen aus Lysin- oder Argininresten besteht oder eine Kombination von Lysin- und Argininresten darstellt, mit Carboxypeptidase B.

2. Verfahren nach Anspruch 1, worin das Peptid oder Protein, dargestellt durch die Formel (II), eine Vorstufe von menschlichem Calcitonin ist.

3. Verfahren zur Herstellung von menschlichem Calcitonin, dargestellt durch die Formel
R-NH₂
worin die NH₂-Gruppe von der α-amidierten C-Terminus Aminosäure des Calcitoninmoleküls abstammt, das das Behandeln eines Peptids oder Proteins der Formel
R-Gly
worin Gly einen C-Terminus Glycinrest darstellt, mit einem C-Terminus-α-amidierenden Enzym vom menschlichen Schilddrüsenursprung oder einem Derivat davon umfaßt.

4. Verfahren zur Herstellung eines Peptids oder Proteins, dargestellt durch die Formel (III):
R-X-NH₂ (III)
worin X-NH₂ eine C-Terminus-α-amidierte Aminosäure darstellt und R einen restlichen Teil des Peptids oder Proteins darstellt, das die Schritte umfaßt:
(1) Hydrolyse eines Peptids oder Proteins, dargestellt durch die Formel (I):
R-X-Gly-B (I)
worin X einen beliebigen Aminosäurerest darstellt, Gly einen Glycinrest darstellt, B einen Lysin- oder Argininrest, ein Oligopeptid, das im wesentlichen aus Lysin- oder Argininresten besteht oder eine Kombination aus Lysin- und Argininresten darstellt und R einen restlichen Teil des Peptids oder Proteins darstellt, mit Carboxypeptidase B, um ein Intermediatpeptid oder -protein zu erzeugen, dargestellt durch die Formel (II):
R-X-Gly (II)
worin X einen beliebigen Aminosäurerest, der α-amidiert werden soll, darstellt, Gly einen C-Terminus Glycinrest darstellt und R einen restlichen Teil des Peptids oder Proteins darstellt; und
(2) Behandeln des Intermediatpeptids oder-proteins, dargestellt durch die Formel (II), mit einem C-Terminus-α-amidierenden Enzym oder einem Derivat davon.

5. Verfahren nach Anspruch 4, worin das durch die Formel (III) dargestellte Peptid oder Protein menschliches Calcitonin ist.

6. Verfahren nach Anspruch 4, worin das C-Terminus-α-amidierende Enzym vom Xenopus laevis- oder von menschlichem Schilddrüsen-Ursprung ist.

7. Verfahren zur Herstellung eines C-Terminus-α-amidierenden Enzyms oder eines Derivats davon, das die Schritte umfaßt:
Kultivierung tierischer Zellen, die mit einem Plasmid transfektiert sind, das eine DNA-Codierung für das Enzym oder einem Derivat davon enthält und dieses exprimieren kann; sowie
Aufarbeitung des Enzyms oder eines Derivats davon.

8. Verfahren nach Anspruch 7, worin das C-Terminus-α-amidierende Enzym XA457 mit einer Aminosäuresequenz ist, die mit der 37. Aminosäure beginnt und mit der 363. Aminosäure endet, dargestellt in Fig. 13-1 bis 13-3.

9. Verfahren nach Anspruch 7, worin das Derivat des C-Terminus-α-amidierenden Enzyms XA799 BglII mit einer Aminosäuresequenz ist, die mit der 39. Aminosäure beginnt und mit der 692. Aminosäure endet, dargestellt in den Fig. 14-1 bis 14-3, sowie einem C-Terminus Leucin.

10. Verfahren nach Anspruch 7, worin die transfektierten tierischen Zellen CHO dhfr(-) Zellen sind, die mit einem Plasmid transfektiert sind, der einen SV40 Promotor, eine DNA-Codierung für eine Aminosäuresequenz, die mit der 39. Aminosäure beginnt und mit der 692. Aminosäure endet, dargestellt in den Fig. 14-1 bis 14-3 und einen C-Terminus Leucin umfaßt, wobei die DNA mit dem SV40 Promotor operabel verbunden ist, und darüberhinaus ein dhfr-Gen umfaßt, und die transfektierten tierischen Zellen durch eine sequentielle Kultivierung in Medien, die Methatorexat in bis zu 3 »M steigender Konzentration enthalten, einer Genamplifikation unterzogen werden, um das Enzymderivat in einer Menge von 2.000 Einheiten/ml Überstand herzustellen.

11. Verfahren zur Herstellung eines Peptids oder Proteins, dargestellt durch die Formel (II):
R-X-Gly (II)
worin X einen beliebigen Aminosäurerest darstellt, Gly einen C-Terminus Glycinrest darstellt und R einen restlichen Teil eines Peptids oder Proteins darstellt, das die Schritte umfaßt:
(1) Erhalten eines kondensierten Proteins, dargestellt durch die Formel (0):
P-R-X-Gly-B (0)
worin P ein Partnerprotein des kondensierten Proteins darstellt, X einen beliebigen Aminosäurerest darstellt; Gly einen Glycinrest dartellt, B einen C-Terminus Lysin- oder Argininrest oder ein im wesentlichen aus Lysin- oder Argininresten bestehendes Oligopeptid oder eine Kombination von Lysin- und Argininresten darstellt und R einen restlichen Teil des Proteins darstellt, durch Kultivierung eines Wirtes, der mit einem Expressionsvektor transformiert wurde, der einen DNA-Codierung für das kondensierte Protein enthält;
(2) Aufarbeitung des kondensierten Proteins;
(3) Spaltung des kondensierten Proteins durch ein konventionelles Verfahren, um ein Partnerprotein (P) und ein Peptid oder Protein zu bilden, dargestellt durch die Formel (I):
R-X-Gly-B (I)
worin R, X, Gly und B die gleiche Bedeutung wie unter Formel (0) definiert haben;
(4) Abtrennen des Peptids oder Proteins (I) vom Partnerprotein (P) auf der Basis der Differenz deren isoelektrischer Punkte; und
(5) Hydrolyse des Peptids oder Proteins (I) mit Carboxypeptidase B, um das gewünschte Peptid oder Protein (II) zu bilden.

12. Verfahren nach Anspruch 11, worin das durch die Formel (II) dargestellte Peptid oder Protein eine Vorstufe von menschlichem Calcitonin ist.

13. Verfahren zur Herstellung eines Peptids oder Proteins, dargestellt durch die Formel (III):
R-X-NH₂ (III)
worin X-NH₂ eine C-Terminus-α-amidierte Aminosäure darstellt und R einen restlichen Teil des Peptids oder Proteins darstellt, das die Schritte umfaßt:
(1) Herstellen eines kondensierten Proteins, dargestellt durch die Formel (0):
P-R-X-Gly-B (0)
worin P ein Partnerprotein des kondensierten Proteins darstellt, X einen beliebigen Aminosäurerest darstellt; Gly einen Glycinrest dartellt, B einen C-Terminus Lysin- oder Argininrest oder ein im wesentlichen aus Lysin- oder Argininresten bestehendes Oligopeptid oder eine Kombination von Lysin- und Argininresten darstellt und R einen restlichen Teil des Proteins darstellt, durch Kultivieren eines Wirtes, der mit einem Expressionsvektor transformiert wurde, der eine DNA-Codierung für das kondensierte Protein enthält;
(2) Aufarbeitung des kondensierten Proteins;
(3) Spalten des kondensierten Proteins durch ein konventionelles Verfahren, um ein Partnerprotein (P) und ein Peptid oder Protein zu bilden, dargestellt durch die Formel (I):
R-X-Gly-B (I)
worin R, X, Gly und B die gleichen Bedeutungen wie unter der Formel (0) definiert haben;
(4) Abtrennen des Peptids oder Proteins (I) vom Partnerprotein (P) auf der Basis der Differenz deren isoelektrischer Punkte; und
(5) Hydrolyse des Peptids oder Proteins (I) mit Carboxypeptidase B, um ein Intermediatpeptid oder - protein zu bilden, dargestellt durch die Formel (II):
R-X-Gly (II)
worin X einen beliebigen Aminosäurerest darstellt, Gly einen C-Terminus Glycinrest darstellt und R einen restlichen Teil eines Peptids oder Proteins darstellt; und
(6) Behandeln des Peptids oder Proteins (II) mit einem C-Terminus-α-amidierenden Enzym oder einem Derivat davon.

14. Verfahren nach Anspruch 13, worin das durch die Formel (III) dargestellte Peptid oder Protein menschliches Calcitonin ist.

## Patentansprüche (Patentansprüche für folgende(n) Vertragsstaat(en): DK)

1. Verfahren zur Herstellung eines Peptids oder Proteins dargestellt durch die Formel (II):
R-X-Gly (II)
worin X einen beliebigen Aminosäurerest darstellt, Gly einen C-Terminus Glycinrest darstellt und R einen restlichen Teil des Peptids oder Proteins darstellt, das die Schritte umfaßt:
Hydrolyse eines Peptids oder Proteins, dargestellt durch die Formel (I):
R-X-Gly-B
worin X einen beliebigen Aminosäurerest, Gly einen Glycinrest, B einen Lysin- oder Argininrest oder ein Oligopeptid, das im wesentlichen aus Lysin- oder Argininresten besteht oder eine Kombination von Lysin- und Argininresten darstellt, mit Carboxypeptidase B.

2. Verfahren nach Anspruch 1, worin das Peptid oder Protein, dargestellt durch die Formel (II), eine Vorstufe von menschlichem Calcitonin ist.

3. Verfahren zur Herstellung von menschlichem Calcitonin, dargestellt durch die Formel
R-NH₂
worin die NH₂-Gruppe von der α-amidierten C-Terminus Aminosäure des Calcitoninmoleküls abstammt, das das Behandeln eines Peptids oder Proteins der Formel
R-Gly
worin Gly einen C-Terminus Glycinrest darstellt, mit einem C-Terminus-α-amidierenden Enzym vom menschlichen Schilddrüsenursprung oder einem Derivat davon umfaßt.

4. Verfahren zur Herstellung eines Peptids oder Proteins, dargestellt durch die Formel (III):
R-X-NH₂ (III)
worin X-NH₂ eine C-Terminus-α-amidierte Aminosäure darstellt und R einen restlichen Teil des Peptids oder Proteins darstellt, das die Schritte umfaßt:
(1) Hydrolyse eines Peptids oder Proteins, dargestellt durch die Formel (I):
R-X-Gly-B (I)
worin X einen beliebigen Aminosäurerest darstellt, Gly einen Glycinrest darstellt, B einen Lysin- oder Argininrest, ein Oligopeptid, das im wesentlichen aus Lysin- oder Argininresten besteht oder eine Kombination aus Lysin- und Argininresten darstellt und R einen restlichen Teil des Peptids oder Proteins darstellt, mit Carboxypeptidase B, um ein Intermediatpeptid oder -Protein zu erzeugen, dargestellt durch die Formel (II):
R-X-Gly (II)
worin X einen beliebigen Aminosäurerest, der α-amidiert werden soll, darstellt, Gly einen C-Terminus Glycinrest darstellt und R einen restlichen Teil des Peptids oder Proteins darstellt; und
(2) Behandeln des Intermediatpeptids oder-proteins, dargestellt durch die Formel (II), mit einem C-Terminus-α-amidierenden Enzym oder einem Derivat davon.

5. Verfahren nach Anspruch 4, worin das durch die Formel (III) dargestellte Peptid oder Protein menschliches Calcitonin ist.

6. Verfahren nach Anspruch 4, worin das C-Terminus-α-amidierende Enzym vom Xenopus laevis- oder von menschlichem Schilddrüsen-Ursprung ist.

7. Verfahren zur Herstellung eines C-Terminus-α-amidierenden Enzyms oder eines Derivats davon, das die Schritte umfaßt:
Kultivierung tierischer Zellen, die mit einem Plasmid transfektiert sind, das eine DNA-Codierung für das Enzym oder einem Derivat davon enthält und dieses exprimieren kann; sowie
Aufarbeitung des Enzyms oder eines Derivats davon.

8. Verfahren nach Anspruch 7, worin das C-Terminus-α-amidierende Enzym XA457 mit einer Aminosäuresequenz ist, die mit der 37. Aminosäure beginnt und mit der 363. Aminosäure endet, dargestellt in Fig. 13-1 bis 13-3.

9. Verfahren nach Anspruch 7, worin das Derivat des C-Terminus-α-amidierenden Enzyms XA799 BglII mit einer Aminosäuresequenz ist, die mit der 39. Aminosäure beginnt und mit der 692. Aminosäure endet, dargestellt in den Fig. 14-1 bis 14-3, sowie einem C-Terminus Leucin.

10. Verfahren nach Anspruch 7, worin die transfektierten tierischen Zellen CHO dhfr(-) Zellen sind, die mit einem Plasmid transfektiert sind, der einen SV40 Promotor, eine DNA-Codierung für eine Aminosäuresequenz, die mit der 39. Aminosäure beginnt und mit der 692. Aminosäure endet, dargestellt in den Fig. 14-1 bis 14-3 und einen C-Terminus Leucin umfaßt, wobei die DNA mit dem SV40 Promotor operabel verbunden ist, und darüberhinaus ein dhfr-Gen umfaßt, und die transfektierten tierischen Zellen durch eine sequentielle Kultivierung in Medien, die Methatorexat in bis zu 3 »M steigender Konzentration enthalten, einer Genamplifikation unterzogen werden, um das Enzymderivat in einer Menge von 2.000 Einheiten/ml Überstand herzustellen.

11. Verfahren zur Herstellung eines Peptids oder Proteins, dargestellt durch die Formel (II):
R-X-Gly (II)
worin X einen beliebigen Aminosäurerest darstellt, Gly einen C-Terminus Glycinrest darstellt und R einen restlichen Teil eines Peptids oder Proteins darstellt, das die Schritte umfaßt:
(1) Erhalten eines kondensierten Proteins, dargestellt durch die Formel (0):
P-R-X-Gly-B (0)
worin P ein Partnerprotein des kondensierten Proteins darstellt, X einen beliebigen Aminosäurerest darstellt; Gly einen Glycinrest dartellt, B einen C-Terminus Lysin- oder Argininrest oder ein im wesentlichen aus Lysin- oder Argininresten bestehendes Oligopeptid oder eine Kombination von Lysin- und Argininresten darstellt und R einen restlichen Teil des Proteins darstellt, durch Kultivierung eines Wirtes, der mit einem Expressionsvektor transformiert wurde, der einen DNA-Codierung für das kondensierte Protein enthält;
(2) Aufarbeitung des kondensierten Proteins;
(3) Spaltung des kondensierten Proteins durch ein konventionelles Verfahren, um ein Partnerprotein (P) und ein Peptid oder Protein zu bilden, dargestellt durch die Formel (I):
R-X-Gly-B (I)
worin R, X, Gly und B die gleiche Bedeutung wie unter Formel (0) definiert haben;
(4) Abtrennen des Peptids oder Proteins (I) vom Partnerprotein (P) auf der Basis der Differenz deren isoelektrischer Punkte; und
(5) Hydrolyse des Peptids oder Proteins (I) mit Carboxypeptidase B, um das gewünschte Peptid oder Protein (II) zu bilden.

12. Verfahren nach Anspruch 11, worin das durch die Formel (II) dargestellte Peptid oder Protein eine Vorstufe von menschlichem Calcitonin ist.

13. Verfahren zur Herstellung eines Peptids oder Proteins, dargestellt durch die Formel (III):
R-X-NH₂ (III)
worin X-NH₂ eine C-Terminus-α-amidierte Aminosäure darstellt und R einen restlichen Teil des Peptids oder Proteins darstellt, das die Schritte umfaßt:
(1) Herstellen eines kondensierten Proteins, dargestellt durch die Formel (0):
P-R-X-Gly-B (0)
worin P ein Partnerprotein des kondensierten Proteins darstellt, X einen beliebigen Aminosäurerest darstellt; Gly einen Glycinrest dartellt, B einen C-Terminus Lysin- oder Argininrest oder ein im wesentlichen aus Lysin- oder Argininresten bestehendes Oligopeptid oder eine Kombination von Lysin- und Argininresten darstellt und R einen restlichen Teil des Proteins darstellt, durch Kultivieren eines Wirtes, der mit einem Expressionsvektor transformiert wurde, der eine DNA-Codierung für das kondensierte Protein enthält;
(2) Aufarbeitung des kondensierten Proteins;
(3) Spalten des kondensierten Proteins durch ein konventionelles Verfahren, um ein Partnerprotein (P) und ein Peptid oder Protein zu bilden, dargestellt durch die Formel (I):
R-X-Gly-B (I)
worin R, X, Gly und B die gleichen Bedeutungen wie unter der Formel (0) definiert haben;
(4) Abtrennen des Peptids oder Proteins (I) vom Partnerprotein (P) auf der Basis der Differenz deren isoelektrischer Punkte; und
(5) Hydrolyse des Peptids oder Proteins (I) mit Carboxypeptidase B, um ein Intermediatpeptid oder - protein zu bilden, dargestellt durch die Formel (II):
R-X-Gly (II)
worin X einen beliebigen Aminosäurerest darstellt, Gly einen C-Terminus Glycinrest darstellt und R einen restlichen Teil eines Peptids oder Proteins darstellt; und
(6) Behandeln des Peptids oder Proteins (II) mit einem C-Terminus-α-amidierenden Enzym oder einem Derivat davon.

14. Verfahren nach Anspruch 13, worin das durch die Formel (III) dargestellte Peptid oder Protein menschliches Calcitonin ist.

15. C-Terminus-α-amidierendes Enzym, erhältlich durch das Verfahren nach Anspruch 7, mit einer Aminosäuresequenz, die mit der 37. Aminosäure beginnt und an der 363. Aminosäure endet, gezeigt in den Fig. 13-1 bis 13-3.

16. C-Terminus-α-amidierendes Enzymderivat, erhältlich durch das Verfahren nach Anspruch 7, mit einer Aminosäuresequenz, die an der 39. Aminosäure beginnt und an der 692. Aminosäure endet, gezeigt in Fig. 14-1 bis 14-3.

## Revendications (Revendications pour l'(les) Etat(s) contractant(s) suivant(s): AT, BE, CH, DE, ES, FR, GB, IT, LI, LU, NL, SE)

1. Un procédé de production d'un peptide ou d'une protéine représenté par la formule (II):
R-X-Gly (II)
dans laquelle
X représente tout résidu d'acide aminé,
Gly représente un résidu glycine en position C-terminale, et
R représente une portion restante du peptide ou de la protéine,
comprenant les étapes d'hydrolyse d'un peptide ou d'une protéine représentée par la formule (I):
R-X-Gly-B
dans laquelle
X représente tout résidu d'acide aminé,
Gly représente un résidu glycine,
B représente un résidu lysine ou arginine, un oligopeptide consistant essentiellement en des résidus lysine ou arginine ou une combinaison de résidus lysine ou arginine,
avec la carboxypeptidase B.

2. Un procédé selon la revendication 1, dans lequel le peptide ou la protéine représenté par la formule 2 est un précurseur de calcétonine humaine.

3. Un procédé de production de calcitonine humaine représentée par la formule
R-NH₂
dans laquelle le groupe -NH₂ est celui d'un acide aminé C-terminal α-amidé de la molécule de calcitonine,
comprenant le traitement d'un peptide ou d'une protéine de formule
R-Gly
dans laquelle Gly représente un résidu glycine en position C-terminale
avec une enzyme d'α-amidation en position C-terminale de la glande thyroïde humaine ou d'un dérivé de cette dernière.

4. Un procédé de production d'un peptide ou d'une protéine représenté par la formule (III):
R-X-NH₂ (III)
dans laquelle
X-NH₂ représente un acide aminé d'α-amidation en position C-terminale et
R représente une partie restante du peptide ou de la protéine,
comprenant les étapes de:
(1) hydrolyse d'un peptide ou d'une protéine représenté par la formule (I):
R-X-Gly-B (I)
dans laquelle
X représente tout résidu d'acide aminé,
Gly représente un résidu glycine,
B représente un résidu lysine ou arginine, un oligopeptide comportant essentiellement des résidus lysine ou arginine ou une combinaison de résidus lysine et arginine, et
R représente une partie restante du peptide ou de la protéine,
avec la carboxypeptidase B pour produire un peptide intermédiaire ou une protéine intermédiaire représenté par la formule (II):
R-X-Gly (II)
dans laquelle
X représente tout résidu d'acide aminé devant être α-amidé,
Gly représente un résidu glycine en position C-terminale et
R représente une partie restante du peptide et de la protéine; et
(2) traitement de la protéine ou du peptide intermédiaire représenté par la formule (II) avec une enzyme d'α-amidation en position C-terminale ou un dérivé de cette dernière.

5. Un procédé selon la revendication 5, dans lequel le peptide ou la protéine représenté par la formule (III) est la calcitonine humaine.

6. Un procédé selon la revendication 4, dans lequel l'enzyme d'α-amidation en position C-terminale provient de Xenopus laevis ou de la thyroïde humaine.

7. Un procédé de production d'une enzyme d'α-amidation en position C-terminale ou d'un dérivé de cette dernière comprenant une étape de culture de cellules animales transfectées avec un plasmide contenant un ADN codant pour ladite enzyme ou un dérivé de cette dernière, et capable d'exprimer cette dernière et une étape de récupération de l'enzyme ou du dérivé de cette dernière.

8. Un procédé selon la revendication 7, dans lequel l'enzyme d'α-amidation en position C-terminale est XA457 comportant une séquence d'acide aminé commençant à l'acide aminé -37 et se terminant à l'acide aminé 363 représenté sur les figures 13-1 à 13-3.

9. Un procédé selon la revendication 7, dans lequel le dérivé de l'enzyme d'α-amidation en position C-terminale est XA799 BglII comprenant une séquence d'acides aminés commençant à l'acide aminé -39 et terminant à l'acide aminé 692 représentés sur les figures 14-1 à 14-3, et un résidu leucine en C terminal.

10. Un procédé selon la revendication 7, dans lequel les cellules animales transfectées sont des cellules CHO dhfr(-) transfectées par un plasmide comprenant un promoteur SV40, un ADN codant pour une séquence d'acide aminé commençant à l'acide aminé -39 et se terminant à l'acide aminé 692 représentés sur les figures 14-1 à 14-3, et un résidu leucine C terminal, l'ADN étant lié de façon fonctionnelle avec le promoteur de SV40, et comprenant de plus un gène dhfr et les cellules animales transfectées étant soumises à une amplification de gène par une culture séquentielle dans des milieux contenant du méthatorexate en des concentrations croissantes allant jusqu'à 3mM, pour produire le dérivé d'enzyme en une quantité de 2000 unités/ml de surnageant.

11. un procédé de production d'un peptide ou d'une protéine représenté par la formule (II)
R-X-Gly (II)
dans laquelle
X représente tout résidu d'acide aminé,
Gly représente un résidu glycine en position C-terminale et
R représente une partie restante du peptide de la protéine,
comprenant les étapes de:
(1) obtention d'une protéine de fusion représentée par la formule (0):
P-R-X-Gly-B (0)
dans laquelle
P représente une protéine associée de la protéine de fusion,
X représente tout résidu d'acide aminé,
Gly représente un résidu glycine,
B représente un résidu lysine ou arginine en position C-terminale ou un oligopeptide consistant essentiellement en des résidus lysine ou arginine ou une combinaison de résidus lysine et arginine et
R représente une partie restante de la protéine,
par culture d'un hôte transformé avec un vecteur d'expression contenant un ADN codant pour la protéine de fusion;
(2) récupération de la protéine de fusion;
(3) clivage de la protéine de fusion par une méthode classique pour former une protéine associée (P) et un peptide ou une protéine représenté par la formule (I):
R-X-Gly-B (I)
dans laquelle R, X, Gly et B sont tels que définis dans la formule (0);
(4) séparation du peptide ou d'une protéine (I) de la protéine associée (P) sur la base de la différence de leurs points isoélectriques; et
(5) hydrolyse du peptide ou de la protéine (I) par la carboxypeptidase B pour former le peptide ou la protéine désiré (II).

12. Un procédé selon la revendication 11, dans lequel la protéine représentée par la formule (II) est un précurseur de la calcitonine humaine.

13. Un procédé de production d'un peptide ou d'une protéine représenté par la formule (III):
R-X-NH₂ (III)
dans laquelle
X-NH₂ représente un acide aminé α-amidé en position C-terminale et
R représente une partie restante de la protéine ou du peptide
comprenant les étapes de:
(1) obtention d'une protéine de fusion représentée par la formule (0):
P-R-X-Gly-B (0)
dans laquelle
P représente une protéine associée de la protéine de fusion,
X représente tout résidu d'acide aminé,
Gly représente un résidu glycine,
B représente un résidu lysine ou arginine en position C-terminale ou un oligopeptide consistant essentiellement en des résidus lysine ou arginine ou une combinaison de résidus lysine et arginine et
R représente une partie restante de la protéine,
par culture d'un hôte transformé avec un vecteur d'expression contenant un ADN codant pour la protéine de fusion;
(2) récupération de la protéine de fusion;
(3) clivage de la protéine de fusion par une méthode classique pour former une protéine associée (P) et un peptide ou une protéine représenté par la formule (I):
R-X-Gly-B (I)
dans laquelle R, X, Gly et B sont tels que définis dans la formule (0);
(4) séparation du peptide ou d'une protéine (I) de la protéine associée (P) sur la base de la différence de leurs points isoélectriques; et
(5) hydrolyse du peptide ou de la protéine (I) par la carboxypeptidase B pour former une protéine ou un peptide intermédiaire représenté par la formule (II).
R-X-Gly (II)
dans laquelle
X représente tout résidu d'acide aminé,
Gly représente un résidu glycine C-terminal et
R représente une partie restante du peptide ou de la protéine; et
(6) traitement du peptide ou de la protéine (II) avec une enzyme d'α-amidation en position C-terminale ou un dérivé de cette dernière.

14. Un procédé selon la revendication 13, dans lequel la peptide ou la protéine représentée par la formule (III) est la calcitonine humaine.

## Revendications (Revendications pour l'(les) Etat(s) contractant(s) suivant(s): DK)

1. Un procédé de production d'un peptide ou d'une protéine représenté par la formule (II):
R-X-Gly (II)
dans laquelle
X représente tout résidu d'acide aminé,
Gly représente un résidu glycine en position C-terminale,et
R représente une portion restante du peptide ou de la protéine,
comprenant les étapes d'hydrolyse d'un peptide ou d'une protéine représentée par la formule (I):
R-X-Gly-B
dans laquelle
X représente tout résidu d'acide aminé,
Gly représente un résidu glycine,
B représente un résidu lysine ou arginine, un oligopeptide consistant essentiellement en des résidus lysine ou arginine ou une combinaison de résidus lysine ou arginine,
avec la carboxypeptidase B.

2. Un procédé selon la revendication 1, dans lequel le peptide ou la protéine représenté par la formule 2 est un précurseur de calcétonine humaine.

3. Un procédé de production de calcitonine humaine représentée par la formule
R-NH₂
dans laquelle le groupe -NH₂ est celui d'un acide aminé C-terminal α-amidé de la molécule de calcitonine,
comprenant le traitement d'un peptide ou d'une protéine de formule
R-Gly
avec une enzyme d'α-amidation en position C-terminale de la glande thyroïde humaine ou d'un dérivé de cette dernière.

4. Un procédé de production d'un peptide ou d'une protéine représenté par la formule (III):
R-X-NH₂ (III)
dans laquelle
X-NH₂ représente un acide aminé d'α-amidation en position C-terminale et
R représente une partie restante du peptide ou de la protéine,
comprenant les étapes de:
(1) hydrolyse d'un peptide ou d'une protéine représenté par la formule (I):
R-X-Gly-B (I)
dans laquelle
X représente tout résidu d'acide aminé,
Gly représente un résidu glycine,
B représente un résidu lysine ou arginine, un oligopeptide comportant essentiellement des résidus lysine ou arginine ou une combinaison de résidus lysine et arginine, et
R représente une partie restante du peptide ou de la protéine,
avec la carboxypeptidase B pour produire un peptide intermédiaire ou une protéine intermédiaire représenté par la formule (II):
R-X-Gly (II)
dans laquelle
X représente tout résidu d'acide aminé devant être α-amidé,
Gly représente un résidu glycine en position C-terminale et
R représente une partie restante du peptide et de la protéine; et
(2) traitement de la protéine ou du peptide intermédiaire représenté par la formule (II) avec une enzyme d'α-amidation en position C-terminale ou un dérivé de cette dernière.

5. Un procédé selon la revendication 5, dans lequel le peptide ou la protéine représenté par la formule (III) est la calcitonine humaine.

6. Un procédé selon la revendication 4, dans lequel l'enzyme d'α-amidation en position C-terminale provient de Xenopus laevis ou de la thyroïde humaine.

7. Un procédé de production d'une enzyme d'α-amidation en position C-terminale ou d'un dérivé de cette dernière comprenant une étape de culture de cellules animales transfectées avec un plasmide contenant un ADN codant pour ladite enzyme ou un dérivé de cette dernière, et capable d'exprimer cette dernière et une étape de récupération de l'enzyme ou du dérivé de cette dernière.

8. Un procédé selon la revendication 7, dans lequel l'enzyme d'α-amidation en position C-terminale est XA457 comportant une séquence d'acide aminé commençant à l'acide aminé -37 et se terminant à l'acide aminé 363 représenté sur les figures 13-1 à 13-3.

9. Un procédé selon la revendication 7, dans lequel le dérivé de l'enzyme d'α-amidation en position C-terminale est XA799 BglII comprenant une séquence d'acides aminés commençant à l'acide aminé -39 et terminant à l'acide aminé 692 représentés sur les figures 14-1 à 14-3, et un résidu leucine en C terminal.

10. Un procédé selon la revendication 7, dans lequel les cellules animales transfectées sont des cellules CHO dhfr(-) transfectées par un plasmide comprenant un promoteur SV40, un ADN codant pour une séquence d'acide aminé commençant à l'acide aminé -39 et se terminant à l'acide aminé 692 représentés sur les figures 14-1 à 14-3, et un résidu leucine C terminal, l'ADN étant lié de façon fonctionnelle avec le promoteur de SV40, et comprenant de plus un gène dhfr et les cellules animales transfectées étant soumises à une amplification de gène par une culture séquentielle dans des milieux contenant du méthatorexate en des concentrations croissantes allant jusqu'à 3mM, pour produire le dérivé d'enzyme en une quantité de 2000 unités/ml de surnageant.

11. un procédé de production d'un peptide ou d'une protéine représenté par la formule (II)
R-X-Gly (II)
dans laquelle
X représente tout résidu d'acide aminé,
Gly représente un résidu glycine en position C-terminale et
R représente une partie restante du peptide de la protéine,
comprenant les étapes de:
(1) obtention d'une protéine de fusion représentée par la formule (0):
P-R-X-Gly-B (0)
dans laquelle
P représente une protéine associée de la protéine de fusion,
X représente tout résidu d'acide aminé,
Gly représente un résidu glycine,
B représente un résidu lysine ou arginine en position C-terminale ou un oligopeptide consistant essentiellement en des résidus lysine ou arginine ou une combinaison de résidus lysine et arginine et
R représente une partie restante de la protéine,
par culture d'un hôte transformé avec un vecteur d'expression contenant un ADN codant pour la protéine de fusion;
(2) récupération de la protéine de fusion;
(3) clivage de la protéine de fusion par une méthode classique pour former une protéine associée (P) et un peptide ou une protéine représenté par la formule (I):
R-X-Gly-B (I)
dans laquelle R, X, Gly et B sont tels que définis dans la formule (0);
(4) séparation du peptide ou d'une protéine (I) de la protéine associée (P) sur la base de la différence de leurs points isoélectriques; et
(5) hydrolyse du peptide ou de la protéine (I) par la carboxypeptidase B pour former le peptide ou la protéine désiré (II).

12. Un procédé selon la revendication 11, dans lequel la protéine représentée par la formule (II) est un précurseur de la calcitonine humaine.

13. Un procédé de production d'un peptide ou d'une protéine représentée par la formule (III):
R-X-NH₂ (III)
dans laquelle
X-NH₂ représente un acide aminé α-amidé en position C-terminale et
R représente une partie restante de la protéine ou du peptide
comprenant les étapes de:
(1) obtention d'une protéine de fusion représentée par la formule (0):
P-R-X-Gly-B (0)
dans laquelle
P représente une protéine associée de la protéine de fusion,
X représente tout résidu d'acide aminé,
Gly représente un résidu glycine,
B représente un résidu lysine ou arginine à position C-terminale ou un oligopeptide consistant essentiellement en des résidus lysine ou arginine ou une combinaison de résidus lysine et arginine et
R représente une partie restante de la protéine,
par culture d'un hôte transformé avec un vecteur d'expression contenant un ADN codant pour la protéine de fusion;
(2) recupération de la protéine de fusion;
(3) clivage de la protéine de fusion par une méthode classique pour former une protéine associée (P) et un peptide ou une protéine représenté par la formule (I):
R-X-Gly-B (I)
dans laquelle R, X, Gly et B sont tels que définis dans la formule (0);
(4) séparation du peptide ou d'une protéine (I) de la protéine associée (P) sur la base de la différence de leurs points isoélectriques; et
(5) hydrolyse du peptide ou de la protéine (I) par la carboxypeptidase B pour former une protéine ou un peptide intermédiaire représenté par la formule (II).
R-X-Gly (II)
dans laquelle
X représente tout résidu d'acide aminé,
Gly représente un résidu glycine C-terminal et
R représente une partie restante du peptide ou de la protéine; et
(6) traitement du peptide ou de la protéine (II) avec une enzyme d'α-amidation en position C-terminale ou un dérivé de cette dernière.

14. Un procédé selon la revendication 13, dans lequel la peptide ou la protéine représentée par la formule (III) est la calcitonine humaine.

15. Une enzyme d'α-amidation C-terminale susceptible d'être obtenue par le procédé selon la revendication 7, comportant une séquence d'acides aminés commençant à l'acide aminé -37 et se terminant à l'acide aminé 363, représentés sur les figures 13-1 à 13-3.

16. Un dérivé d'enzyme de la famille d'α-amidation C-terminale susceptible d'être obtenu par le procédé selon la revendication 7, et comportant une séquence d'acide aminé commençant à l'acide aminé -39 et se terminant à l'acide aminé 692 représentés sur les figures 14-1 à 14-3.
